(19) Europäisches Patentamt
European Patent Office
Office européen des brevets

(11) EP 3 766 881 A1

(12) # EUROPEAN PATENT APPLICATION
published in accordance with Art. 153(4) EPC

(43) Date of publication:
**20.01.2021 Bulletin 2021/03**

(21) Application number: **19767181.1**

(22) Date of filing: **08.03.2019**

(51) Int Cl.:
***C07D 471/04*** *(2006.01)* ***A01N 43/76*** *(2006.01)*
***A01N 43/90*** *(2006.01)* ***A01N 47/02*** *(2006.01)*
***A01P 7/04*** *(2006.01)* ***A61K 31/506*** *(2006.01)*
***A61P 33/00*** *(2006.01)* ***A61P 33/14*** *(2006.01)*
***C07D 413/04*** *(2006.01)* ***C07D 413/14*** *(2006.01)*
***C07F 7/10*** *(2006.01)*

(86) International application number:
**PCT/JP2019/009418**

(87) International publication number:
**WO 2019/176791 (19.09.2019 Gazette 2019/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2018 JP 2018044222**

(71) Applicant: **Nippon Soda Co., Ltd.**
**Tokyo 100-8165 (JP)**

(72) Inventors:
- **SAKANISHI Keita**
**Odawara-shi, Kanagawa 250-0280 (JP)**
- **AOYAMA Hikaru**
**Odawara-shi, Kanagawa 250-0280 (JP)**
- **SAKIYAMA Norifumi**
**Odawara-shi, Kanagawa 250-0280 (JP)**
- **IWASA Takao**
**Odawara-shi, Kanagawa 250-0280 (JP)**
- **MATSUI Maki**
**Odawara-shi, Kanagawa 250-0280 (JP)**
- **KOBAYASHI Tomomi**
**Odawara-shi, Kanagawa 250-0280 (JP)**
- **KITAYAMA Takashi**
**Odawara-shi, Kanagawa 250-0280 (JP)**

(74) Representative: **Mewburn Ellis LLP**
**Aurora Building**
**Counterslip**
**Bristol BS1 6BX (GB)**

(54) # HETEROARYL PYRIMIDINE COMPOUND AND PEST CONTROL AGENT

(57) The present invention relates to a compound represented by the following Formula (I) or a salt thereof, which exhibits superior insecticidal activity, acaricidal activity, and/or nematicidal activity, which exhibits superior safety, and which can be advantageously synthesized industrially, and also relates to a formulation for controlling harmful organisms containing the same as an active ingredient.

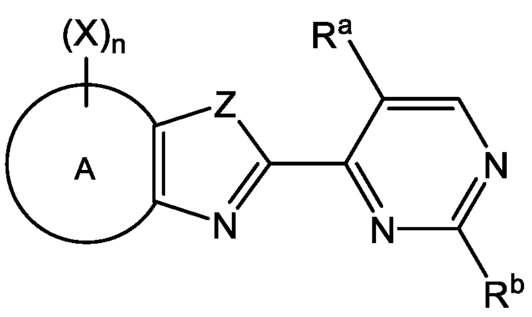

(I)

wherein, X represents a halogeno group, a substituted or unsubstituted C1-6 alkyl group, or the like; n is an integer ranging from 1 to 3; Z represents an oxygen atom, a sulfur atom, or NR; R represents a hydrogen atom or a substituted or unsubstituted C1-6 alkyl group; A represents a benzene ring or a 6-membered heteroaryl ring; $R^a$ represents a

EP 3 766 881 A1

substituted or unsubstituted C1-6 alkylsulfonyl group; and $R^b$ represents a substituted or unsubstituted C1-6 alkyl group, and the like.

**Description**

TECHNICAL FIELD

The present invention relates to a heteroarylpyrimidine compound and a formulation for controlling harmful organisms. More particularly, the present invention relates to a heteroarylpyrimidine compound which has superior insecticidal activity and/or acaricidal activity, exhibits superior safety, and can be advantageously synthesized industrially, and also relates to a formulation for controlling harmful organisms containing the same as an active ingredient.

The present application claims priority on Japanese Patent Application No. 2018-044222, filed in Japan on March 12, 2018, the content of which is incorporated herein by reference.

BACKGROUND ART

Various compounds having an insecticidal and/or acaricidal activity have been proposed. In order to practically use such compounds as agrochemicals, the compounds are required not only to have a sufficient efficacy, but also to hardly cause chemical resistance, avoid phytotoxicity against plants or soil contamination, and have a low level of toxicity against livestock, fish or the like.

Patent Document 1 discloses compounds represented by Formula (A), and the like.

[Chem. 1]

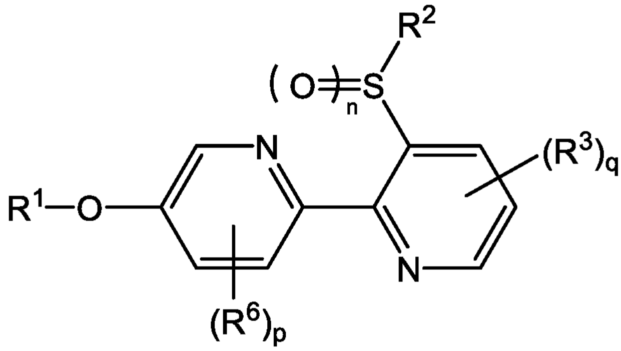

(A)

Patent Document 2 discloses compounds represented by Formula (B), and the like.

[Chem. 2]

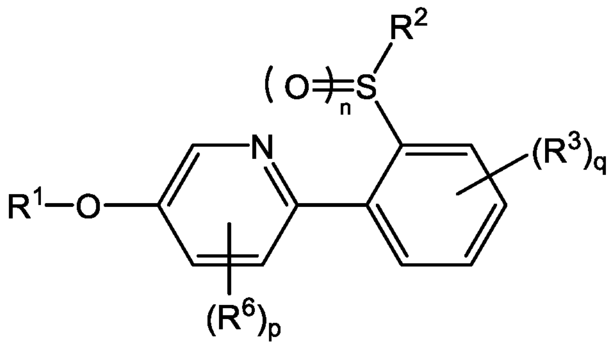

(B)

PRIOR ART LITERATURE

Patent Documents

[Patent Document 1] PCT international Publication No. WO2016/121969 A
[Patent Document 2] PCT international Publication No. WO2017/069105 A

DISCLOSURE OF INVENTION

Technical Problem

An object of the present invention is to provide a heteroarylpyrimidine compound which has superior activity for controlling harmful organisms, and in particular, superior insecticidal activity and/or acaricidal activity, exhibits superior safety, and can be advantageously synthesized industrially. Another object of the present invention is to provide a formulation for controlling harmful organisms, an insecticide or acaricide, a formulation for controlling ectoparasites, or a formulation for controlling or expelling endoparasites, which contains the same heteroarylpyrimidine compound as an active ingredient.

Solution to Problem

As a result of diligent studies in order to achieve the objects mentioned above, the inventors of the present application completed the present invention including the following modes.

[1] A compound represented by Formula (I), an N-oxide compound thereof, a stereoisomer, a tautomer, a hydrate, or a salt thereof.

[Chem. 3]

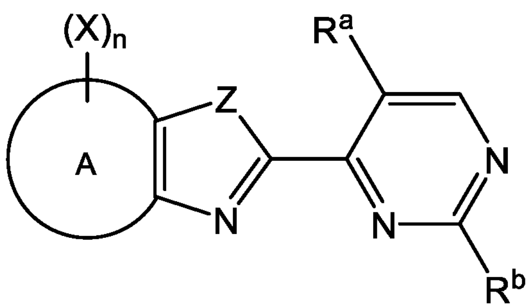

(I)

wherein

X is a halogeno group, a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a nitro group, a cyano group, or a group represented by $-NR^1R^2$,
each of $R^1$ and $R^2$ is independently a hydrogen atom, a substituted or unsubstituted C1-6 alkyl group, a substituted or unsubstituted C1-6 alkyl carbonyl group, or a substituted or unsubstituted C1-6 alkoxy carbonyl group,
n is an integer of any one of 1 to 3,
Z is an oxygen atom, a sulfur atom or NR,
R is a hydrogen atom or a substituted or unsubstituted C1-6 alkyl group,
A is a benzene ring or a 6-membered heteroaryl ring,

$R^a$ is a substituted or unsubstituted C1-6 alkylsulfonyl group,
$R^b$ is a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkyl carbonyl group, a substituted or unsubstituted C1-6 alkoxy carbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a nitro group, a cyano group, a group represented by $-NR^3R^4$, a group represented by $-C(=O)NR^8R^9$, a group represented by $-C(=NH)R^{10}$, a group represented by $-CR^{11}=N-OR^{12}$, a group represented by-$C(=O)N=S(O)_mR^{13}$, or a group represented by $-N=S(O)R^{14}R^{15}$,
each of $R^3$ and $R^4$ is independently a hydrogen atom, a substituted or unsubstituted C1-6 alkyl group, a substituted or unsubstituted C1-6 alkyl carbonyl group, a substituted or unsubstituted C1-6 alkoxy carbonyl group, an amino group, a group represented by $-C(=O)NR^5R^6$, or a group represented by $-C(=NH)R^7$,
each of $R^5$ and $R^6$ is independently a hydrogen atom, or a substituted or unsubstituted C1-6 alkyl group,
$R^7$ is a C1-6 alkoxy group, or an amino group,
each of $R^8$ and $R^9$ is independently a hydrogen atom, or a substituted or unsubstituted C1-6 alkyl group,
$R^{10}$ is a C1-6 alkoxy group, or an amino group,
each of $R^{11}$ and $R^{12}$ is independently a hydrogen atom, or a substituted or unsubstituted C1-6 alkyl group,
m is 0 or 1,
$R^{13}$ is a C1-6 alkyl group, and
each of $R^{14}$ and $R^{15}$ is a C1-6 alkyl group.

[2] A formulation for controlling harmful organisms, containing at least one compound selected from the group consisting of the compounds as recited in [1] mentioned above, N-oxide compounds thereof, stereoisomers, tautomers, hydrates, and salts thereof as an active ingredient.
[3] An insecticidal formulation or an acaricidal formulation, containing at least one compound selected from the group consisting of the compounds as recited in [1] mentioned above, N-oxide compounds thereof, stereoisomers, tautomers, hydrates, and salts thereof as an active ingredient.
[4] A formulation for controlling ectoparasites, containing at least one compound selected from the group consisting of the compounds as recited in [1] mentioned above, N-oxide compounds thereof, stereoisomers, tautomers, hydrates, and salts thereof as an active ingredient.
[5] A formulation for controlling endoparasites or for expelling endoparasites, containing at least one compound selected from the group consisting of the compounds as recited in [1] mentioned above, N-oxide compounds thereof, stereoisomers, tautomers, hydrates, and salts thereof as an active ingredient.

Advantageous Effects of the Invention

The heteroarylpyrimidine compounds of the present invention can control harmful organisms which are problematic in view of farm products or for hygiene reasons. In particular, the compounds can effectively control various types of agricultural pests and acari with a reduced concentration. In addition, the heteroarylpyrimidine compounds of the present invention can effectively control ectoparasites and endoparasites which harm humans and animals.

EMBODIMENTS OF THE INVENTION

[Heteroarylpyrimidine compounds]

A heteroarylpyrimidine compound of the present invention is a compound represented by Formula (I) (hereinafter, referred to as compound (I) in some cases), an N-oxide compound thereof, a tautomer thereof, a hydrate thereof, or a salt of compound (I). The compound represented by Formula (I) contains all the stereoisomers which are enantiomers or diastoreomers.
In the present invention, the term "unsubstituted" means that only a group which is a mother nucleus is present. When only the name of a group as a mother nucleus is described without the term "substituted", it means "unsubstituted" unless otherwise specified.
On the other hand, the term "substituted (= having a substituent)" means that at least one hydrogen atom of a group as a mother nucleus is substituted with a group (substituent) having a structure which is the same as or different from the mother nucleus. Therefore, a "substituted group (substituent)" is another group which is bonded to the group as the mother nucleus. The substituted group may be one, or may be two or more. Two or more substituted groups may

be the same as or different from each other.

The term "C1-6" represents that the number of carbon atoms of a group as a mother nucleus is 1 to 6. The number of carbon atoms does not include the number of carbon atoms present in a substituted group. For example, a butyl group having an ethoxy group as a substituted group is classified as a C2 alkoxy C4 alkyl group.

A "substituted group" is not particularly limited as long as it is chemically acceptable and has the effect of the present invention. Hereinafter, as examples of a group which can be a "substituted group", mention may be made of,

a C1-6 alkyl group such as a methyl group, an ethyl group, an n-propyl group, an i-propyl group, an n-butyl group, an s-butyl group, an i-butyl group, a t-butyl group, an n-pentyl group, or an n-hexyl group;

a C2-6 alkenyl group such as a vinyl group, a 1-propenyl group, a 2-propenyl group (an allyl group), a 1-butenyl group, a 2-butenyl group, a 3-butenyl group, a 1-methyl-2-propenyl group, or a 2-methyl-2-propenyl group;

a C2-6 alkynyl group such as an ethynyl group, a 1-propynyl group, a 2-propynyl group, a 1-butynyl group, a 2-butynyl group, a 3-butynyl group, or a 1-methyl-2-propynyl group;

a C3-8 cycloalkyl group such as a cyclopropyl group, a cyclobutyl group, a cyclopentyl group, a cyclohexyl group, or a cubanyl group;

a C6-10 aryl group such as a phenyl group, or a naphthyl group;

a C6-10 aryl C1-6 alkyl group such as a benzyl group, or a phenethyl group;

a 3- to 6-membered heterocyclyl group;

a 3- to 6-membered heterocyclyl C1-6 alkyl group;

a hydroxyl group;

a C1-6 alkoxy group such as a methoxy group, an ethoxy group, an n-propoxy group, an i-propoxy group, an n-butoxy group, an s-butoxy group, an i-butoxy group, or a t-butoxy group;

a C2-6 alkenyloxy group such as a vinyloxy group, an allyloxy group, a propenyloxy group, or a butenyloxy group;

a C2-6 alkynyloxy group such as an ethynyloxy group, or a propargyloxy group;

a C6-10 aryloxy group such as a phenoxy group, or a naphthoxy group;

a C6-10 aryl C1-6 alkoxy group such as a benzyloxy group, or a phenethyloxy group;

a 5- to 6-membered heteroaryloxy group such as a thiazolyloxy group, or a pyridyloxy group;

a 5- to 6-membered heteroaryl C1-6 alkyloxy group such as a thiazolyl methyloxy group, or a pyridyl methyloxy group;

a formyl group;

a C1-6 alkyl carbonyl group such as an acetyl group, or a propionyl group;

a formyloxy group;

a C1-6 alkyl carbonyloxy group such as an acetyloxy group, or a propionyloxy group;

a C6-10 aryl carbonyl group such as a benzoyl group;

a C1-6 alkoxy carbonyl group such as a methoxycarbonyl group, an ethoxycarbonyl group, an n-propoxycarbonyl group, an i-propoxycarbonyl group, an n-butoxycarbonyl group, or a t-butoxycarbonyl group;

a C1-6 alkoxy carbonyloxy group such as a methoxycarbonyloxy group, an ethoxycarbonyloxy group, an n-propoxycarbonyloxy group, an i-propoxycarbonyloxy group, an n-butoxycarbonyloxy group, or a t-butoxycarbonyloxy group;

a carboxyl group;

a halogeno group such as a fluoro group, a chloro group, a bromo group, or an iodo group;

a C1-6 haloalkyl group such as a chloromethyl group, a chloroethyl group, a trifluoromethyl group, a 1,2-dichloro-n-propyl group, a 1-fluoro-n-butyl group, or a perfluoro-n-pentyl group;

a C2-6 haloalkenyl group such as a 2-chloro-1-propenyl group, or a 2-fluoro-1-butenyl group;

a C2-6 haloalkynyl group such as a 4,4-dichloro-1-butynyl group, a 4-fluoro-1-pentynyl group, or a 5-bromo-2-pentynyl group;

a C1-6 haloalkoxy group such as a trifluoromethoxy group, a 2-chloro-n-propoxy group, or a 2,3-dichlorobutoxy group;

a C2-6 haloalkenyloxy group such as a 2-chloropropenyloxy group, or a 3-bromobutenyloxy group;

a C1-6 haloalkyl carbonyl group such as a chloroacetyl group, a trifluoroacetyl group, or a trichloroacetyl group;

an amino group;

a C1-6 alkyl substituted amino group such as a methylamino group, a dimethylamino group, or a diethylamino group;

a C6-10 aryl amino group such as an anilino group, or a naphthyl amino group;

a C6-10 aryl C1-6 alkyl amino group such as a benzylamino group, or a phenethyl amino group;

a formylamino group;

a C1-6 alkyl carbonylamino group such as an acetylamino group, a propanoylamino group, a butyrylamino group, or an i-propyl carbonyl amino group;

a C1-6 alkoxy carbonyl amino group such as a methoxycarbonyl amino group, an ethoxycarbonyl amino group, an n-propoxycarbonyl amino group, or an i-propoxycarbonyl amino group;

a substituted or unsubstituted aminocarbonyl group such as an aminocarbonyl group, a dimethyl aminocarbonyl group, a phenyl aminocarbonyl group, or an N-phenyl-N-methyl aminocarbonyl group;

an imino C1-6 alkyl group such as an iminomethyl group, a (1-imino)ethyl group, or a (1-imino)-n-propyl group;

a substituted or unsubstituted N-hydroxyimino C1-6 alkyl group such as an N-hydroxy-iminomethyl group, a (1-(N-hydroxy)-imino)ethyl group, a (1-(N-hydroxy)-imino)propyl group, an N-methoxy-iminomethyl group, or a (1-(N-methoxy)-imino)ethyl group;
an aminocarbonyloxy group;
a C1-6 alkyl-substituted aminocarbonyloxy group such as an ethyl aminocarbonyloxy group, or a dimethyl aminocarbonyloxy group;
a mercapto group;
a C1-6 alkylthio group such as a methylthio group, an ethylthio group, an n-propylthio group, an i-propylthio group, an n-butylthio group, an i-butylthio group, an s-butylthio group, or a t-butylthio group;
a C1-6 haloalkylthio group such as a trifluoromethylthio group, or a 2,2,2-trifluoroethylthio group;
a C6-10 arylthio group such as a phenylthio group, or a naphthylthio group;
a 5- to 6-membered heteroarylthio group such as a thiazolylthio group, or a pyridylthio group;
a C1-6 alkylsulfinyl group such as a methylsulfinyl group, an ethylsulfinyl group, or a t-butylsulfinyl group;
a C1-6 haloalkylsulfinyl group such as a trifluoromethyl sulfinyl group, or a 2,2,2-trifluoroethyl sulfinyl group;
a C6-10 arylsulfinyl group such as a phenylsulfinyl group;
a 5- to 6-membered heteroarylsulfinyl group such as a thiazolylsulfinyl group, or a pyridylsulfinyl group;
a C1-6 alkylsulfonyl group such as a methylsulfonyl group, an ethylsulfonyl group, or a t-butylsulfonyl group;
a C1-6 haloalkylsulfonyl group such as a trifluoromethyl sulfonyl group, or a 2,2,2-trifluoroethyl sulfonyl group;
a C6-10 arylsulfonyl group such as a phenylsulfonyl group;
a 5- to 6-membered heteroaryl sulfonyl group such as a thiazolylsulfonyl group, or a pyridylsulfonyl group;
a C1-6 alkylsulfonyloxy group such as a methylsulfonyloxy group, an ethylsulfonyloxy group, or a t-butylsulfonyloxy group;
a C1-6 haloalkyl sulfonyloxy group such as a trifluoromethyl sulfonyloxy group, or a 2,2,2-trifluoroethyl sulfonyloxy group;
a tri C1-6 alkyl-substituted silyl group such as a trimethylsilyl group, a triethylsilyl group, or a t-butyldimethylsilyl group;
a tri C6-10 aryl-substituted silyl group such as a triphenylsilyl group;
a cyano group; and
a nitro group.

In addition, any of the hydrogen atoms in these "substituted groups" may be substituted with other substituted groups having a different structure. In this case, as examples of the "substituted groups", mention may be made of a C1-6 alkyl group, a C1-6 haloalkyl group, a C1-6 alkoxy group, a C1-6 haloalkoxy group, a halogeno group, a cyano group, a nitro group and the like.

In addition, the aforementioned "3- to 6-membered heterocyclyl group" is a group having 1 to 4 heteroatoms selected from the group consisting of a nitrogen atom, an oxygen atom and a sulfur atom as a constitutional atom of the ring. The heterocyclyl group may be a monocyclyl group or a polycyclyl group. As long as at least one ring is a hetero ring in the polyheterocyclyl group, the remaining ring may be a saturated alicyclic ring, an unsaturated alicyclic ring or an aromatic ring. As examples of the "3-to 6-membered heterocyclyl group", mention may be made of a 3- to 6-membered saturated heterocyclyl group, a 5- to 6-membered heteroaryl group, a 5-to 6-membered partially unsaturated heterocyclyl group, and the like.

As examples of the "3- to 6-membered saturated heterocyclyl group", mention may be made of an aziridinyl group, an epoxy group, a pyrrolidinyl group, a tetrahydrofuranyl group, a thiazolidinyl group, a piperidinyl group, a piperazinyl group, a morpholinyl group, a dioxolanyl group, a dioxanyl group, and the like.

As examples of the "5-membered heteroaryl group", mention may be made of a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group, and the like.

As examples of the "6-membered heteroaryl group", mention may be made of a pyridyl group, a pyrazinyl group, a pyrimidinyl group, a pyridazinyl group, a triazinyl group and the like.

[X]

In Formula (I), X is a halogeno group, a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a nitro group, a cyano group, or a group represented by $-NR^1R^2$.

As examples of the "halogeno group" of X, mention may be made of a fluoro group, a chloro group, a bromo group, an iodo group, and the like.

The "C1-6 alkyl group" of X may be linear or branched. As examples of the alkyl group, mention may be made

of a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, an i-hexyl group, and the like.

As examples of the preferable substituted group on the "C1-6 alkyl group" in X, mention may be made of a halogeno group such as a fluoro group, a chloro group, a bromo group, or an iodo group; a C1-6 alkoxy group such as a methoxy group, an ethoxy group, an n-propoxy group, an i-propoxy group, an n-butoxy group, an s-butoxy group, an i-butoxy group, or a t-butoxy group; a C1-6 haloalkoxy group such as 2-chloro-n-propoxy group, a 2,3-dichlorobutoxy group, or a trifluoromethoxy group; a cyano group; a 2-trimethylsilylethoxymethyl group; and the like.

As examples of the preferable "substituted C1-6 alkyl group", mention may be made of a C1-6 haloalkyl group such as a chloromethyl group, a chloroethyl group, a trifluoromethyl group, a pentafluoroethyl group, a 1,2-dichloro-n-propyl group, a 1,1,1,3,3,3-hexafluoropropan-2-yl group, a perfluoropropan-2-yl group, a 1-fluoro-n-butyl group, a perfluoro-n-pentyl group, or the like.

As examples of the "C2-6 alkenyl group" of X, mention may be made of a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group, and the like.

As examples of the "C2-6 alkynyl group" of X, mention may be made of an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, a 1,1-dimethyl-2-butynyl group, and the like.

As examples of the "C1-6 alkoxy group" of X, mention may be made of a methoxy group, an ethoxy group, an n-propoxy group, an n-butoxy group, an n-pentyloxy group, an n-hexyloxy group, an i-propoxy group, an i-butoxy group, an s-butoxy group, a t-butoxy group, an i-hexyloxy group, and the like.

As examples of the "C1-6 alkylthio group" of X, mention may be made of a methylthio group, an ethylthio group, an n-propylthio group, an n-butylthio group, an n-pentylthio group, an n-hexylthio group, an i-propylthio group, and the like.

As examples of the "C1-6 alkylsulfinyl group" of X, mention may be made of a methylsulfinyl group, an ethylsulfinyl group, a t-butylsulfinyl group, and the like.

As examples of the "C1-6 alkylsulfonyl group" of X, mention may be made of a methylsulfonyl group, an ethylsulfonyl group, a t-butylsulfonyl group, and the like.

As examples of the preferable substituted groups on the "C2-6 alkenyl group", "C2-6 alkynyl group", "C1-6 alkoxy group", "C1-6 alkylthio group", "C1-6 alkylsulfinyl group", and "C1-6 alkylsulfonyl group" of X, mention may be made of a halogeno group such as a fluoro group, a chloro group, a bromo group, or an iodo group; a hydroxyl group; a C1-6 alkoxy group such as a methoxy group, an ethoxy group, an n-propoxy group, an i-propoxy group, an n-butoxy group, an s-butoxy group, an i-butoxy group, or a t-butoxy group; a C1-6 haloalkoxy group such as a 2-chloro-n-propoxy group, a 2,3-dichlorobutoxy group, or a trifloromethoxy group; and a cyano group. As examples of the more preferable substituted groups, mention may be made of a halogeno group such as a fluoro group, a chloro group, a bromo group, or an iodo group; and a hydroxyl group.

As examples of the "C3-8 cycloalkyl group" of X, mention may be made of a cyclopropyl group, a cyclobutyl group, a cyclopentyl group, a cyclohexyl group, or a cycloheptyl group.

The "C6-10 aryl group" of X may be a monocyclic aryl group or a polycyclic aryl group. As long as the polycyclic aryl group has at least one aromatic ring, the remaining rings may be any ones of a saturated alicyclic ring, an unsaturated alicyclic ring and an aromatic ring.

As examples of the "C6-10 aryl group", mention may be made of a phenyl group, a naphthyl group, an azulenyl group, an indenyl group, an indanyl group, a tetralinyl group, and the like.

As examples of the "5-membered heteroaryl group" of X, mention may be made of a pyrrolyl group, a furyl group, a thienyl group, an imidazolyl group, a pyrazolyl group, an oxazolyl group, an isoxazolyl group, a thiazolyl group, an isothiazolyl group, a triazolyl group (specifically, a [1,2,3]triazolyl group or a [1,2,4]triazolyl group), an oxadiazolyl group (specifically, a [1,2,3]oxadiazolyl group, a [1,2,4]oxadiazolyl group, a [1,2,5]oxadiazolyl group, or a [1,3,4]oxadiazolyl group), a thiadiazolyl group, a tetrazolyl group, and the like. As examples of the "6-membered heteroaryl group", mention may be made of a pyridyl group, a pyrazinyl group, a pyrimidinyl group, a pyridazinyl group, a triazinyl group, and the like.

As examples of the "C6-10 aryloxy group" of X, mention may be made of a phenoxy group, a naphthoxy group, an azulenyloxy group, an indenyloxy group, an indanyloxy group, a tetranyloxy group, and the like.

As examples of the "5- to 6-membered heteroaryloxy group" of X, mention may be made of a thiazolyloxy group, a pyridyloxy group, and the like.

As examples of the preferable substituted groups on the "C3-8 cycloalkyl group", "C6-10 aryl group", "heteroaryl group", "C6-10 aryloxy group", and "5- to 6-membered heteroaryloxy group" of X, mention may be made of a halogeno

group such as a fluoro group, a chloro group, a bromo group, or an iodo group; a C1-6 alkyl group such as a methyl group, an ethyl group, an n-propyl group, an i-propyl group, an n-butyl group, an s-butyl group, an i-butyl group, a t-butyl group, an n-pentyl group, or an n-hexyl group; a C1-6 haloalkyl group such as a chloromethyl group, a chloroethyl group, a trifluoromethyl group, a 2,2,2-trifluoroethyl group, a pentafluoroethyl group, a 1,2-dichloro-n-propyl group, a 1,1,1,3,3,3-hexafluoropropan-2-yl group, a perfluoropropan-2-yl group, a 1-fluoro-n-butyl group, or a perfluoro-n-pentyl group; a C1-6 alkoxy group such as a methoxy group, an ethoxy group, an n-propoxy group, an i-propoxy group, an n-butoxy group, an s-butoxy group, an i-butoxy group, or a t-butoxy group; a C1-6 haloalkoxy group such as a 2-chloro-n-propoxy group, a 2,3-dichlorobutoxy group, or a trifloromethoxy group; a C1-6 alkyl carbonyl group such as an acetyl group, or a propionyl group; a C1-6 alkoxy carbonyl group such as a methoxycarbonyl group, an ethoxycarbonyl group, an n-propoxycarbonyl group, an i-propoxycarbonyl group, an n-butoxycarbonyl group, or a t-butoxycarbonyl group; a C6-10 aryl carbonyl group such as a benzoyl group; a C1-6 alkylsulfonyl group such as a methylsulfonyl group, an ethylsulfonyl group, or a t-butylsulfonyl group; an amino group; a cyano group; and a pentafluorosulfanyl group. As examples of more preferable substituted groups, mention may be made of a halogeno group, a C1-6 alkyl group, a C1-6 haloalkyl group, and an amino group.

Each of $R^1$ and $R^2$ in the "group represented by $-NR^1R^2$" of X is independently a hydrogen atom, a substituted or unsubstituted C1-6 alkyl group, a substituted or unsubstituted C1-6 alkyl carbonyl group, or a substituted or unsubstituted C1-6 alkoxy carbonyl group.

As examples of the "C1-6 alkyl group" of $R^1$ and $R^2$, mention may be made of the same ones as those listed in X.

As examples of the "C1-6 alkyl carbonyl group" of $R^1$ and $R^2$, mention may be made of an acetyl group, a propionyl group, and the like.

As examples of the "C1-6 alkoxy carbonyl group" of $R^1$ and $R^2$, mention may be made of a methoxycarbonyl group, an ethoxycarbonyl group, an n-propoxycarbonyl group, an i-propoxycarbonyl group, a t-butoxycarbonyl group, and the like.

As examples of the preferable substituted groups on the "C1-6 alkyl group", "C1-6 alkyl carbonyl group", and "C1-6 alkoxy carbonyl group" of $R^1$ and $R^2$, mention may be made of a halogeno group such as a fluoro group, a chloro group, a bromo group, or an iodo group, or a cyano group.

In Formula (I), n represents the number of X substituted on A, and is an integer of any one of 1 to 3. When n is 2 or 3, the two or three Xs may be the same as or different from each other.

As examples of the preferable X, mention may be made of a C1-6 haloalkylthio group such as a trifluoromethylthio group or a 2,2,2-trifluoroethylthio group; a C1-6 haloalkylsulfinyl group such as a trifluoromethylsulfinyl group, or a 2,2,2-trifluoroethylsulfinyl group; a C1-6 haloalkylsulfonyl group such as a trifluoromethylsulfonyl group, or a 2,2,2-trifluoroethylsulfonyl group; a C1-6 alkyl group; a C1-6 haloalkyl group; and the like.

[$R^a$]

In Formula (I), $R^a$ is a substituted or unsubstituted C1-6 alkylsulfonyl group.

As examples of the "C1-6 alkylsulfonyl group" of $R^a$, mention may be made of a methylsulfonyl group, an ethylsulfonyl group, a t-butylsulfonyl group, and the like.

As preferable examples of the substituted group on the "C1-6 alkylsulfonyl group" of $R^a$, mention may be made of a halogeno group such as a fluoro group, a chloro group, a bromo group, or an iodo group; a C1-6 alkoxy group such as a methoxy group, an ethoxy group, an n-propoxy group, an i-propoxy group, an n-butoxy group, an s-butoxy group, an i-butoxy group, or a t-butoxy group; a C1-6 haloalkoxy group such as a 2-chloro-n-propoxy group, a 2,3-dichlorobutoxy group, or a trifloromethoxy group; and a cyano group.

As preferable examples of $R^a$, mention may be made of an ethylsulfonyl group and the like.

[$R^b$]

In Formula (I), $R^b$ is a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkyl carbonyl group, a substituted or unsubstituted C1-6 alkoxy carbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a nitro group, a cyano group, a group represented by $-NR^3R^4$, a group represented by $-C(=O)NR^8R^9$, a group represented by $-C(=NH)R^{10}$, a group represented by $-CR^{11}=N-OR^{12}$, a group represented by $-C(=O)N=S(O)_mR^{13}$, or a group represented by $-N=S(O)R^{14}R^{15}$.

As examples of the substituted groups on the "C1-6 alkyl group", "C2-6 alkenyl group", "C2-6 alkynyl group",

"C1-6 alkoxy group", "C1-6 alkylthio group", "C1-6 alkylsulfinyl group", "C1-6 alkylsulfonyl group", "C3-8 cycloalkyl group", "C6-10 aryl group", "5- to 6-membered heteroaryl group", "C6-10 aryloxy group, "5- to 6-membered heteroaryloxy group", and the groups thereof used in $R^b$, mention may be made of the same ones as those listed in X.

As examples of the preferable substituted groups of the "C3-8 cycloalkyl group", "C6-10 aryl group", "heteroaryl group", "C6-10 aryloxy group", and "5- to 6-membered heteroaryloxy group" of $R^b$, mention may be made of a C1-6 alkyl group such as a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, or an i-hexyl group; a C1-6 haloalkyl group such as a chloromethyl group, a chloroethyl group, a trifluoromethyl group, a 2,2,2-trifluoroethyl group, a pentafluoroethyl group, a 1,2-dichloro-n-propyl group, a 1,1,1,3,3,3-hexafluoropropan-2-yl group, a perfluoropropan-2-yl group, a 1-fluoro-n-butyl group, or a perfluoro-n-pentyl group; a C1-6 alkyl carbonyl group such as an acetyl group, or a propionyl group; a C6-10 aryl carbonyl group such as a benzoyl group; a C1-6 alkoxy carbonyl group such as a methoxycarbonyl group, an ethoxycarbonyl group, an n-propoxycarbonyl group, an i-propoxycarbonyl group, an n-butoxycarbonyl group, or a t-butoxycarbonyl group; a C1-6 alkylsulfonyl group such as a methylsulfonyl group, an ethylsulfonyl group, or a t-butylsulfonyl group; an amino group; and a cyano group.

As examples of the "C1-6 alkyl carbonyl group" and "C1-6 alkoxy carbonyl group" of $R^b$, mention may be made of the same ones as those listed in $R^1$.

As examples of the preferable substituted groups on the "C1-6 alkyl carbonyl group" and "C1-6 alkoxy carbonyl group" of $R^b$, mention may be made of a halogeno group such as a fluoro group, a chloro group, a bromo group, or an iodo group.

Each of $R^3$ and $R^4$ of the "group represented by $-NR^3R^4$" of $R^b$ is independently a hydrogen atom, a substituted or unsubstituted C1-6 alkyl group, a substituted or unsubstituted C1-6 alkyl carbonyl group, a substituted or unsubstituted C1-6 alkoxy carbonyl group, an amino group, a group represented by $-C(=O)NR^5R^6$, or a group represented by $-C(=NH)R^7$.

As examples of the "C1-6 alkyl group" of $R^3$ and $R^4$, mention may be made of the same ones as those listed in X. As examples of the "C1-6 alkyl carbonyl group" and "C1-6 alkoxy carbonyl group", mention may be made of the same ones listed in $R^1$.

As examples of the preferable substituted groups on the "C1-6 alkyl group", "C1-6 alkyl carbonyl group" and "C1-6 alkoxy carbonyl group" in $R^3$ and $R^4$, mention may be made of a halogeno group such as a fluoro group, a chloro group, a bromo group, or an iodo group, an amino group, or a cyano group. As examples of the more preferable substituents, an amino group may be mentioned.

Each of $R^5$ and $R^6$ in the "group represented by $-C(=O)NR^5R^6$" of $R^3$ and $R^4$ is independently a hydrogen atom, or a C1-6 alkyl group.

As examples of the "C1-6 alkyl group" of $R^5$ and $R^6$, mention may be made of the same ones as those listed in X.

$R^7$ in the "group represented by $-C(=NH)R^7$" of $R^3$ and $R^4$ is a C1-6 alkoxy group, or an amino group.

As examples of the "C1-6 alkoxy group" of $R^7$, mention may be made of the same ones as those listed in X.

Each of $R^8$ and $R^9$ of the "group represented by $-C(=O)NR^8R^9$" of $R^b$ is independently a hydrogen atom, or a C1-6 alkyl group.

As examples of the "C1-6 alkyl group" of $R^8$ and $R^9$, mention may be made of the same ones as those listed in X.

$R^{10}$ in the "group represented by $-C(=NH)R^{10}$" of $R^b$ is a C1-6 alkoxy group, or an amino group.

As examples of the "C1-6 alkoxy group" in $R^{10}$, mention may be made of the same ones as those listed in X.

Each of $R^{11}$ and $R^{12}$ in the "group represented by $-CR^{11}=N-OR^{12}$" in $R^b$ is independently a hydrogen atom, or a substituted or unsubstituted C1-6 alkyl group.

As examples of the "C1-6 alkyl group" of $R^{11}$ and $R^{12}$, mention may be made of the same ones as those listed in X.

As examples of the preferable substituted groups on the "C1-6 alkyl group" in $R^{11}$ and $R^{12}$, mention may be made of a halogeno group such as a fluoro group, a chloro group, a bromo group, or an iodo group.

m in the "group represented by $-C(=O)N=S(O)_mR^{13}$" in $R^b$ is 0 or 1.

$R^{13}$ in the "group represented by $-C(=O)N=S(O)_mR^{13}$" in $R^b$ is a C1-6 alkyl group.

As examples of the "C1-6 alkyl group" of $R^{13}$, mention may be made of the same ones as those listed in X.

$R^{14}$ and $R^{15}$ in the "group represented by $-N=S(O)R^{14}R^{15}$" in $R^b$ is a C1-6 alkyl group.

As examples of the "C1-6 alkyl group" of $R^{14}$ and $R^{15}$, mention may be made of the same ones as those listed in X.

$R^b$ is preferably a substituted or unsubstituted C1-6 alkoxy group, a substituted or unsubstituted C6-10 aryl group, a substituted or unsubstituted 5- to 6-membered heteroaryl group, a substituted or unsubstituted C6-10 aryloxy group, or a group represented by $-NR^3R^4$, and is more preferably a substituted or unsubstituted C1-6 alkoxy group, a substituted or unsubstituted 5- to 6-membered heteroaryl group, or a group represented by $-NR^3R^4$.

[Z]

In Formula (I), Z is an oxygen atom, a sulfur atom or NR. R is a hydrogen atom or a substituted or unsubstituted C1-6 alkyl group.
As examples of the "C1-6 alkyl group" of R, mention may be made of the same ones as those listed in X.
As examples of the preferable substituted groups on the "C1-6 alkyl group" in R, mention may be made of a halogeno group such as a fluoro group, a chloro group, a bromo group, or an iodo group, or a cyano group.
As the R, a methyl group is preferable.

[A]

In Formula (I), A is a benzene ring or a 6-membered heteroaryl ring. The benzene ring or the 6-membered heteroaryl ring of A is ortho-condensed to a 5-membered ring having N (nitrogen atom) and Z (oxygen atom, sulfur atom or NR) as ring-forming atoms.
As examples of the "6-membered heteroaryl ring" of A, mention may be made of a pyridine ring, a pyrazine ring, a pyrimidine ring, a pyridazine ring, and the like.
An N-oxide compound of compound (I) is a compound in which a nitrogen atom forming a ring on the hetero ring is oxidized. As examples of the hetero ring which can form N-oxide, mention may be made of, for example, a pyridine ring, a condensed ring containing a pyridine ring, and the like.
A salt of compound (I) is not particularly limited as long as the salt is an agriculturally and horticulturally acceptable salt. As examples of the salt of compound (I), mention may be made of a salt of an inorganic acid such as hydrochloric acid, or sulfuric acid; a salt of an organic acid such as acetic acid, or lactic acid; a salt of an alkaline metal such as lithium, sodium, or potassium; a salt of an alkaline earth metal such as calcium, or magnesium; a salt of a transition metal such as iron, or copper; a salt of an organic base such as ammonia, triethylamine, tributylamine, pyridine, or hydrazine; and the like.
The heteroarylpyrimidine compound of the present invention (hereinafter, referred to as "compound of the present invention" in some cases) is not particularly limited by the preparation method thereof. For example, the compound (I), an N-oxide compound thereof, a tautomer thereof, a hydrate, or a salt thereof can be obtained by means of the well-known preparation methods described in the Examples and the like. In addition, the tautomer of compound (I), the hydrate, or the salts thereof can be obtained from the compound (I) by means of well-known methods.
The compound represented by Formula (I) is preferably any one of the compounds represented by Formula (1-1) to Formula (1-3).

[Chem. 4]

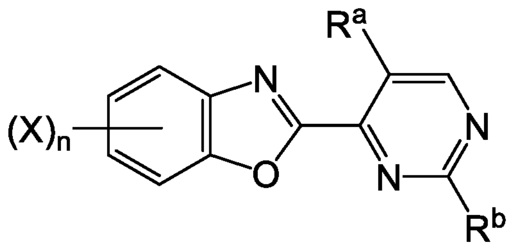

(I-1)

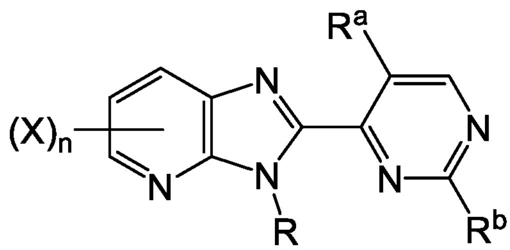

(I-2)

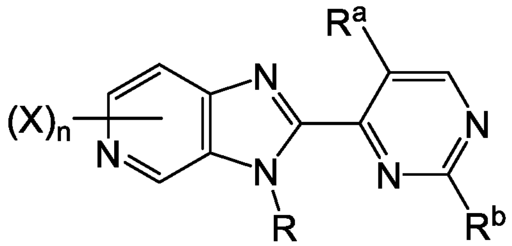

(I-3)

X, n, $R^a$ and $R^b$ in Formula (I-1) have the same meanings of those of Formula (I). X, n, R, $R^a$ and $R^b$ in Formula (1-2) and Formula (1-3) have the same meanings of those of Formula (I).

The compound of the present invention has a superior effect for controlling harmful organisms such as various agricultural pests affecting the plant growth, and acari.

In addition, the compound of the present invention has a reduced phytotoxicity against plants and has a low level of toxicity against fish or warm-blooded animals, and for this reason, the compound of the present invention is a compound with high safety. For this reason, the compound of the present invention is useful as an active ingredient of a pesticide or an acaricide.

In addition, in recent years, many pests such as diamondback moths, planthoppers, leafhoppers and aphids have developed a resistance to various types of conventional agrochemicals, and for this reason, a problem occurs in which the efficacy of the conventional agrochemicals has become insufficient. Therefore, agrochemicals that are effective even for the resistant strains of pests are desired. The compounds of the present invention exhibit superior effects for controlling not only the sensitive strains of pests, but also various resistant strains of pests and acaricide-resistant strains of acari.

The compounds of the present invention have a superior effect for controlling the ectoparasites and endoparasites harmful for humans and animals. In addition, the compounds of the present invention have a low level of toxicity to the fish or warm-blooded animals, and for this reason, the heteroarylpyrimidine compounds are highly safe compounds. For this reason, the compounds of the present invention are useful as an active ingredient of a formulation for controlling ectoparasites and endoparasites.

In addition, the compounds of the present invention are effective for controlling the targeted organisms in any development stages, and exhibit superior effects of controlling, for example, acari and insects in the stages of eggs, nymph, larvae, pupae and adults.

[Formulation for controlling harmful organisms, insecticide, or acaricide]

The formulation for controlling harmful organisms, the insecticide, or the acaricide of the present invention contains at least one compound selected from the heteroarylpyrimidine compounds of the present invention as an active ingredient. The amount of the heteroarylpyrimidine compound contained in the formulation for controlling harmful organisms, the insecticide, or the acaricide of the present invention is not particularly limited as long as an effect of controlling harmful organisms, agricultural pests, or acari is exhibited.

The formulation for controlling harmful organisms, the insecticide, or the acaricide of the present invention is preferably used for crops; vegetables; edible roots; tuber crops; flowers; fruit trees; trees of tea, coffee, cacao or foliage plants; grasses for pastures; grasses for lawns; plants such as cotton; or the like.

As for the application to the plants, the formulation for controlling harmful organisms, the insecticide or the acaricide of the present invention may be applied on any one part of the plants, such as leaf, stem, stalk, flower, bud,

fruit, seed, sprout, root, tuber, tuberous root, shoot, cutting and the like.

In addition, the plant varieties for which the formulation for controlling harmful organisms, the insecticide or the acaricide of the present invention is applicable are not particularly limited. As examples of the plant varieties, mention may be made of originals, varieties, improved varieties, cultivated varieties, mutant plants, hybrid plants, genetically modified organisms (GMO) and the like.

The formulations for controlling harmful organisms of the present invention can be used for controlling various agricultural pests and acari by seed treatment, foliar spraying, soil application, water surface application and the like.

Specific examples of the various agricultural pests and acari which can be controlled by the formulations for controlling harmful organisms of the present invention are shown below.

(1) Lepidoptera Butterflies and Moths

(a) Arctiidae moths, for example, Hyphantria cunea and Lemyra imparilis;
(b) Bucculatricidae moths, for example, Bucculatrix pyrivorella;
(c) Carposinidae, for example, Carposina sasakii;
(d) Crambidae moths, for example, Diaphania indica and Diaphania nitidalis of Diaphania spp.; Ostrinia furnacalis, Ostrinia nubilalis and Ostrinia scapulalis of Ostrinia spp.; and others such as Chilo suppressalis, Cnaphalocrocis medinalis, Conogethes punctiferalis, Diatraea grandiosella, Glyphodes pyloalis, Hellula undalis and Parapediasia teterrella;
(e) Gelechiidae moths, for example, Helcystogramma triannulella, Pectinophora gossypiella, Phthorimaea operculella and Sitotroga cerealella;
(f) Geometridae moths, for example, Ascotis selenaria;
(g) Gracillariidae moths, for example, Caloptilia theivora, Phyllocnistis citrella and Phyllonorycter ringoniella;
(h) Hesperiidae butterflies, for example, Parnara guttata;
(i) Lasiocampidae moths, for example, Malacosoma neustria;
(j) Lymantriidae moths, for example, Lymantria dispar and Lymantria monacha of Lymantria spp.; and others such as Euproctis pseudoconspersa and Orgyia thyellina;
(k) Lyonetiidae moths, for example, Lyonetia clerkella and Lyonetia prunifoliella malinella of Lyonetia spp.;
(l) Noctuidae moths, for example, Spodoptera depravata, Spodoptera eridania, Spodoptera exigua, Spodoptera frugiperda, Spodoptera littoralis and Spodoptera litura of Spodoptera spp.; Autographa gamma and Autographa nigrisigna of Autographa spp.; Agrotis ipsilon and Agrotis segetum of Agrotis spp.; Helicoverpa armigera, Helicoverpa assulta and Helicoverpa zea of Helicoverpa spp.; Heliothis armigera and Heliothis virescens of Heliothis spp.; and others such as Aedia leucomelas, Ctenoplusia agnata, Eudocima tyrannus, Mamestra brassicae, Mythimna separata, Naranga aenescens, Panolis japonica, Peridroma saucia, Pseudoplusia includens and Trichoplusiani;
(m) Nolidae moths, for example, Earias insulana;
(n) Pieridae butterflies, for example, Pieris brassicae and Pieris rapae crucivora of Pieris spp.;
(o) Plutellidae moths, for example, Acrolepiopsis sapporensis and Acrolepiopsis suzukiella of Acrolepiopsis spp.; and others such as Plutella xylostella;
(p) Pyralidae moths, for example, Cadra cautella, Elasmopalpus lignosellus, Etiella zinckenella and Galleria mellonella;
(q) Sphingidae moths, for example, Manduca quinquemaculata and Manduca sexta of Manduca spp.;
(r) Stathmopodidae moths, for example, Stathmopoda masinissa;
(s) Tineidae moths, for example, Tinea translucens;
(t) Tortricidae moths, for example, Adoxophyes honmai and Adoxophyes orana of Adoxophyes spp.; Archips breviplicanus and Archips fuscocupreanus of Archips spp.; and others such as Choristoneura fumiferana, Cydia pomonella, Eupoecilia ambiguella, Grapholitha molesta, Homona magnanima, Leguminivora glycinivorella, Lobesia botrana, Matsumuraeses phaseoli, Pandemis heparana and Sparganothis pilleriana; and
(u) Yponomeutidae moths, for example, Argyresthia conjugella.

(2) Thysanoptera Insect Pests

(a) Phlaeothripidae, for example, Ponticulothrips diospyrosi; and
(b) Thripidae, for example, Frankliniella intonsa and Frankliniella occidentalis of Frankliniella spp.; Thrips palmi and Thrips tabaci of Thrips spp.; and others such as Heliothrips haemorrhoidalis and Scirtothrips dorsalis.

(3) Hemiptera Insect Pests

(A) Archaeorrhyncha

(a) Delphacidae, for example, Laodelphax striatella, Nilaparvata lugens, Perkinsiella saccharicida and Sogatella furcifera.

(B) Clypeorrhyncha

(a) Cicadellidae, for example, Empoasca fabae, Empoasca nipponica, Empoasca onukii and Empoasca sakaii of Empoasca spp.; and others such as Arboridia apicalis, Balclutha saltuella, Epiacanthus stramineus, Macrosteles striifrons and Nephotettix cinctinceps.

(C) Heteroptera

(a) Alydidae, for example, Riptortus clavatus;
(b) Coreidae, for example, Cletus punctiger and Leptocorisa chinensis;
(c) Lygaeidae, for example, Blissus leucopterus, Cavelerius saccharivorus and Togo hemipterus;
(d) Miridae, for example, Halticus insularis, Lygus lineolaris, Psuedatomoscelis seriatus, Stenodema sibiricum, Stenotus rubrovittatus and Trigonotylus caelestialium;
(e) Pentatomidae, for example, Nezara antennata and Nezara viridula of Nezara spp.; Eysarcoris aeneus, Eysarcoris lewisi and Eysarcoris ventralis of Eysarcoris spp.; and others such as Dolycoris baccarum, Eurydema rugosum, Glaucias subpunctatus, Halyomorpha halys, Piezodorus hybneri, Plautia crossota and Scotinophora lurida;
(f) Pyrrhocoridae, for example, Dysdercus cingulatus;
(g) Rhopalidae, for example, Rhopalus msculatus;
(h) Scutelleridae, for example, Eurygaster integriceps; and
(i) Tingidae, for example, Stephanitis nashi.

(D) Sternorrhyncha

(a) Adelgidae, for example, Adelges laricis;
(b) Aleyrodidae, for example, Bemisia argentifolii and Bemisia tabaci of Bemisia spp.; and others such as Aleurocanthus spiniferus, Dialeurodes citri and Trialeurodes vaporariorum;
(c) Aphididae, for example, Aphis craccivora, Aphis fabae, Aphis forbesi, Aphis gossypii, Aphis pomi, Aphis sambuci and Aphis spiraecola of Aphis spp.; Rhopalosiphum maidis and Rhopalosiphum padi of Rhopalosiphum spp.; Dysaphis plantaginea and Dysaphis radicola of Dysaphis spp.; Macrosiphum avenae and Macrosiphum euphorbiae of Macrosiphum spp.; Myzus cerasi, Myzus persicae and Myzus varians of Myzus spp.; and others such as Acyrthosiphon pisum, Aulacorthum solani, Brachycaudus helichrysi, Brevicoryne brassicae, Chaetosiphon fragaefolii, Hyalopterus pruni, Hyperomyzus lactucae, Lipaphis erysimi, Megoura viciae, Metopolophium dirhodum, Nasonovia ribis-nigri, Phorodon humuli, Schizaphis graminum, Sitobion avenae and Toxoptera aurantii;
(d) Coccidae, for example, Ceroplastes ceriferus and Ceroplastes rubens of Ceroplastes spp.;
(e) Diaspididae, for example, Pseudaulacaspis pentagona and Pseudaulacaspis prunicola of Pseudaulacaspis spp.; Unaspis euonymi and Unaspis yanonensis of Unaspis spp.; and others such as Aonidiella aurantii, Comstockaspis perniciosa, Fiorinia theae and Pseudaonidia paeoniae;
(f) Margarodidae, for example, Drosicha corpulenta and Icerya purchasi;
(g) Phylloxeridae, for example, Viteus vitifolii;
(h) Pseudococcidae, for example, Planococcus citri and Planococcus kuraunhiae of Planococcus spp.; and others such as Phenacoccus solani and Pseudococcus comstocki; and
(i) Psyllidae, for example, Psylla mali and Psylla pyrisuga of Psylla spp.; and others such as Diaphorina citri.

(4) Polyphaga Insect Pests

(a) Anobiidae, for example, Lasioderma serricorne;
(b) Attelabidae, for example, Byctiscus betulae and Rhynchites heros;
(c) Bostrichidae, for example, Lyctus brunneus;
(d) Brentidae, for example, Cylas formicarius;
(e) Buprestidae, for example, Agrilus sinuatus;
(f) Cerambycidae, for example, Anoplophora malasiaca, Monochamus alternatus, Psacothea hilaris and Xylotrechus pyrrhoderus;
(g) Chrysomelidae, for example, Bruchus pisorum and Bruchus rufimanus of Bruchus spp.; Diabrotica barberi, Diabrotica undecimpunctata and Diabrotica virgifera of Diabrotica spp.; Phyllotreta nemorum and Phyllotreta striolata of Phyllotreta spp.; and others such as Aulacophora femoralis, Callosobruchus chinensis, Cassida nebulosa, Chaetocnema concinna, Leptinotarsa decemlineata, Oulema oryzae and Psylliodes angusticollis;
(h) Coccinellidae, for example, Epilachna varivestis and Epilachna vigintioctopunctata of Epilachna spp.;
(i) Curculionidae, for example, Anthonomus grandis and Anthonomus pomorum of Anthonomus spp.; Sitophilus granarius and Sitophilus zeamais of Sitophilus spp.; and others such as Echinocnemus squameus, Euscepes postfasciatus, Hylobius abietis, Hypera postica, Lissohoptrus oryzophilus, Otiorhynchus sulcatus, Sitona lineatus and Sphenophorus venatus;
(j) Elateridae, for example, Melanotus fortnumi and Melanotus tamsuyensis of Melanotus spp.;
(k) Nitidulidae, for example, Epuraea domina;
(l) Scarabaeidae, for example, Anomala cuprea and Anomala rufocuprea of Anomala spp.; and others such as Cetonia aurata, Gametis jucunda, Heptophylla picea, Melolontha and Popillia japonica;
(m) Scolytidae, for example, Ips typographus;
(n) Staphylinidae, for example, Paederus fuscipes;
(o) Tenebrionidae, for example, Tenebrio molitor and Tribolium castaneum; and (p) Trogossitidae, for example, Tenebroides mauritanicus.

(5) Diptera Insect Pests

(A) Brachycera

(a) Agromyzidae, for example, Liriomyza bryoniae, Liriomyza chinensis, Liriomyza sativae and Liriomyza trifolii of Liriomyza spp.; and others such as Chromatomyia horticola and Agromyza oryzae;
(b) Anthomyiidae, for example, Delia platura and Delia radicum of Delia spp.; and others such as Pegomya cunicularia;
(c) Drosophilidae, for example, Drosophila melanogaster and Drosophila suzukii of Drosophila spp.;
(d) Ephydridae, for example, Hydrellia griseola;
(e) Psilidae, for example, Psila rosae; and
(f) Tephritidae, for example, Bactrocera cucurbitae and Bactrocera dorsalis of Bactrocera spp.; Rhagoletis cerasi and Rhagoletis pomonella of Rhagoletis spp.; and others such as Ceratitis capitata and Dacus oleae.

(B) Nematocera

(a) Cecidomyiidae, for example, Asphondylia yushimai, Contarinia sorghicola, Mayetiola destructor and Sitodiplosis mosellana.

(6) Orthoptera Insect Pests

(a) Acrididae, for example, Schistocerca americana and Schistocerca gregaria of Schistocerca spp.; and others such as Chortoicetes terminifera, Dociostaurus maroccanus, Locusta migratoria, Locustana pardalina, Nomadacris septemfasciata and Oxya yezoensis;
(b) Gryllidae, for example, Acheta domestica and Teleogryllus emma;
(c) Gryllotalpidae, for example, Gryllotalpa orientalis; and
(d) Tettigoniidae, for example, Tachycines asynamorus.

(7) Acari

(A) Acaridida of Astigmata

(a) Acaridae mites, for example, Rhizoglyphus echinopus and Rhizoglyphus robini of Rhizoglyphus spp.; Tyrophagus neiswanderi, Tyrophagus perniciosus, Tyrophagus putrescentiae and Tyrophagus similis of Tyrophagus spp.; and others such as Acarus siro, Aleuroglyphus ovatus and Mycetoglyphus fungivorus;

(B) Actinedida of Prostigmata

(a) Tetranychidae mites, for example, Bryobia praetiosa and Bryobia rubrioculus of Bryobia spp.; Eotetranychus asiaticus, Eotetranychus boreus, Eotetranychus celtis, Eotetranychus geniculatus, Eotetranychus kankitus, Eotetranychus pruni, Eotetranychus shii, Eotetranychus smithi, Eotetranychus suginamensis and Eotetranychus uncatus of Eotetranychus spp.; Oligonychus hondoensis, Oligonychus ilicis, Oligonychus karamatus, Oligonychus mangiferus, Oligonychus orthius, Oligonychus perseae, Oligonychus pustulosus, Oligonychus shinkajii and Oligonychus ununguis of Oligonychus spp.; Panonychus citri, Panonychus mori and Panonychus ulmi of Panonychus spp.; Tetranychus cinnabarinus, Tetranychus evansi, Tetranychus kanzawai, Tetranychus ludeni, Tetranychus quercivorus, Tetranychus phaselus, Tetranychus urticae and Tetranychus viennensis of Tetranychus spp.; Aponychus corpuzae and Aponychus firmianae of Aponychus spp.; Sasanychus akitanus and Sasanychus pusillus of Sasanychus spp.; Shizotetranychus celarius, Shizotetranychus longus, Shizotetranychus miscanthi, Shizotetranychus recki and Shizotetranychus schizopus of Shizotetranychus spp.; and others such as Tetranychina harti, Tuckerella pavoniformis and Yezonychus sapporensis;

(b) Tenuipalpidae mites, for example, Brevipalpus lewisi, Brevipalpus obovatus, Brevipalpus phoenicis, Brevipalpus russulus and Brevipalpus californicus of Brevipalpus spp.; Tenuipalpus pacificus and Tenuipalpus zhizhilashviliae of Tenuipalpus spp.; and others such as Dolichotetranychus floridanus;

(c) Eriophyidae mites, for example, Aceria diospyri, Aceria ficus, Aceria japonica, Aceria kuko, Aceria paradianthi, Aceria tiyingi, Aceria tulipae and Aceria zoysiea of Aceria spp.; Eriophyes chibaensis and Eriophyes emarginatae of Eriophyes spp.; Aculops lycopersici and Aculops pelekassi of Aculops spp.; Aculus fockeui and Aculus schlechtendali of Aculus spp.; and others such as Acaphylla theavagrans, Calacarus carinatus, Colomerus vitis, Calepitrimerus vitis, Epitrimerus pyri, Paraphytoptus kikus, Paracalacarus podocarpi and Phyllocotruta citri;

(d) Tarsonemidae mites, for example, Tarsonemus bilobatus and Tarsonemus waitei of Tarsonemus spp.; and others such as Phytonemus pallidus and Polyphagotarsonemus latus; and

(e) Penthaleidae mites, for example, Penthaleus erythrocephalus and Penthaleus major of Penthaleus spp.

The formulation for controlling harmful organisms of the present invention may be mixed or used in combination with other active constituents such as fungicides, insecticides / acaricides, nematicides and soil pesticides; and/or plant regulators, synergists, fertilizers, soil conditioners and animal feed.

Combinations of the compound of the present invention with other active constituents can be expected to provide synergistic effects in terms of insecticidal / acaricidal / nematicidal activity. The synergistic effect can be confirmed in accordance with a conventional method by means of an equation defined by Colby (Colby. S. R.; Calculating Synergistic and Antagonistic Responses of Herbicide Combinations; Weeds, 15, pages 20 - 22, 1967).

Examples of the insecticides / acaricides, nematocides, soil pesticides, vermicides and the like which can be mixed or used together with the formulation for controlling harmful organisms according to the present invention are described below.

(1) Acetylcholine esterase inhibitor:

(a) Carbamate-based agents: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, cloethocarb, metam-sodium, and promecarb; and

(b) Organic phosphorus-based agents: acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion; bromophos-ethyl, BRP, carbophenothion, cyanofenphos, CYAP, demeton-S-methyl sulfone, dialifos, dichlofenthion, dioxabenzofos, etrimfos, fensulfothion, flupyrazofos, fonofos, formothion, fosmethilan, isazofos, jodfenphos, methacrifos, pirimiphos-ethyl, phosphocarb, propaphos, prothoate, and sulprofos.

(2) GABA-gated chloride ion channel antagonists: acetoprole, chlordane, endosulfan, ethiprole, fipronil, pyrafluprole, pyriprole, camphechlor, heptachlor, and dienochlor.

(3) Sodium channel modulators: acrinathrin, d-cis-trans-allethrin, d-trans allethrin, bifenthrin, bioallethrin, bioallethrin S-cyclopentyl isomer, bioresmethrin, cycloprothrin, cyfluthrin, β-cyfluthrin, cyhalothrin, λ-cyhalothrin, γ-cyhalothrin, cypermethrin, α-cypermethrin, β-cypermethrin, θ-cypermethrin, ξ-cypermethrin, cyphenothrin [(1R)-trans isomer], δ-methrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethrum, resmethrin, silafluofen, tefluthrin, tetramethrin [(1R)-isomer], tralomethrin, transfluthrin; allethrin, pyrethrins, pyrethrin I, pyrethrin II, profluthrin, dimefluthrin, bioethanomethrin, biopermethrin, transpermethirn, fenfluthrin, fenpirithrin, flubrocythrinate, flufenprox, metofluthrin, protrifenbute, pyresmethrin, and terallethrin.

(4) Nicotinic acetylcholine receptor agonists: acetamiprid, clothianidin, dinotefuran, imidacloprid, nitenpyram, nithiazine, thiacloprid, thiamethoxam, sulfoxaflor, nicotine, flupyradifurone, and flupyrimine.

(5) Nicotinic acetylcholine receptor allosteric modulators: spinetoram, and spinosad.

(6) Chloride channel activators: abamectin, emamectin-benzoate, lepimectin, milbemectin, ivermectin, selamectin, doramectin, eprinomectin, moxidectin, milbemycin; milbemycin oxime, and nemadectin.

(7) Juvenile hormone-like substances: hydroprene, kinoprene, methoprene, fenoxycarb, pyriproxyfen, diofenolan, epofenonane, and triprene.

(8) Other nonspecific inhibitors: methyl bromide, chloropicrin, sulfuryl fluoride, borax, and tartar emetic.

(9) Homoptera selective antifeedants: flonicamid, pymetrozine, and pyrifluquinazon.

(10) Acari growth inhibitors: clofentezine, diflovidazin, hexythiazox, and etoxazole.

(11) Microorganism-derived insect midgut inner membrane distrupting agents: Bacillus thuringiensis subsp. Israelensis, Bacillus sphaericus, Bacillus thuringiensis subsp. Aizawai, Bacillus thuringiensis subsp. Kurstaki, Bacillus thuringiensis subsp. Tenebrionis, Bt crop protein: Cry1Ab, Cry1Ac, Cry1Fa, Cry1A. 105, Cry2Ab, Vip3A, mCry3A, Cry3Ab, Cry3Bb, and Cry34Ab1/Cry35Ab1.

(12) Mitochondria ATP biosynthesis enzyme inhibitors: diafenthiuron, azocyclotin, cyhexatin, fenbutatin oxide, propargite, and tetradifon.

(13) Oxidative phosphorylation decouplers: chlorfenapyr, sulfluramid, DNOC; binapacryl, dinobuton, and dinocap.

(14) Nicotinic acetylcholine receptor channel blockers: bensultap, cartap hydrochloride; nereistoxin; thiosultap-sodium, and thiocyclam.

(15) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezin, and fluazuron.

(16) Diptera molting disruptors: cyromazine.

(17) Molting hormone receptor agonists: chromafenozide, halofenozide, methoxyfenozide, and tebufenozide.

(18) Octopamine receptor agonists: amitraz, demiditraz, and chlordimeform.

(19) Mitochondria electron transfer chain complex III inhibitors: acequinocyl, fluacrypyrim, and hydramethylnon.

(20) Mitochondria electron transfer chain complex I inhibitors: fenazaquin, fenpyroximate, pyrimidifen, pyridaben, tebufenpyrad, tolfenpyrad, and rotenone.

(21) Voltage-dependent sodium channel blockers: indoxacarb, and metaflumizone.

(22) Acetyl CoA carboxylase inhibitors: spirodiclofen, spiromesifen, and spirotetramat.

(23) Mitochondria electron transfer chain complex IV inhibitors: aluminum phosphide, calcium phosphide, phosphine, zinc phosphide, and cyanide.

(24) Mitochondria electron transfer chain complex II inhibitors: cyenopyrafen, cyflumetofen, and pyflubumide.

(25) Ryanodine receptor modulators: chlorantraniliprole, cyantraniliprole, flubendiamide, cyclaniliprole, and tetraniliprole.

(26) Mixed function oxidase inhibitor compounds: piperonyl butoxide.

(27) Latrophilin receptor agonists: depsipeptide, cyclodepsipeptide, 24 membered cyclodepsipeptide, and emodepside.

(28) Others (action mechanism is unknown): azadirachtin, benzoximate, bifenazate, bromopropylate, quinomethionate, cryolite, dicofol, pyridalyl, benclothiaz, sulfur, amidoflumet, 1, 3-dichloropropene, DCIP, phenisobromolate, benzomate, metaldehyde, chlorobenzilate, chlothiazoben, dicyclanil, fenoxacrim, fentrifanil, flubenzimine, fluphenazine, gossyplure, japonilure, metoxadiazone, oil, potassium oleate, tetrasul; triarathene, afidopyropen, flometoquin, flufiprole, fluensulfone, meperfluthrin, tetramethylfluthrin, tralopyril, dimefluthrin, methylneodecanamide; fluralaner, afoxolaner, and fluxametamide, 5-[5-(3,5-dichlorophenyl)-5-trifluoromethyl-4,5-dihydroisoxazol-3-yl]-2-(1H-1,2,4-triazol-1-yl)benzonitrile (CAS: 943137-49-3), broflanilide, other meta-diamides, steinernema carpocapsae, steinernema glaseri, pasteuria penetrans, paecilomyces tenuipes, paecilomyces fumosoroseus, beauveria bassiana, beauveria brongniartii, metarhizium anisopliae, verticillium lecanii, and acynonapyr.

(29) Parasiticide:

(a) Benzimidazole-based agents: fenbendazole, albendazole, triclabendazole, oxibendazole, mebendazole, oxfendazole, parbendazole, flubendazole, febantel, netobimin, thiophanate; thiabendazole, and cambendazole;

(b) Salicylanilide-based agents: closantel, oxyclozanide, rafoxanide, and niclosamide;
(c) Substituted phenol-based agents: nitroxinil, and nitroscanate;
(d) Pyrimidine-based agents: pyrantel, and morantel;
(e) Imidazothiazole-based agents: levamisole, and tetramisole;
(f) Tetrahydropyrimidine-based agents: praziquantel, and epsiprantel; and
(g) Other antiparasitic agents: cyclodien, ryania, clorsulon, metronidazole, demiditraz, piperazine, diethylcarbamazine, dichlorophen, monepantel, tribendimidine, amidantel, thiacetarsamide, melarsomine, and arsenamide.

Specific examples of the fungicides which can be mixed or used together with the formulation for controlling harmful organisms according to the present invention are described below.

(1) Nucleic acid biosynthesis inhibitors:

(a) RNA polymerase I inhibitors: benalaxyl, benalaxyl-M, furalaxyl, metalaxyl, metalaxyl-M, oxadixyl; clozylacon, and ofurace;
(b) Adenosine deaminase inhibitors: bupirimate, dimethirimol, and ethirimol;
(c) DNA/RNA synthesis inhibitors: hymexazol, and octhilinone; and
(d) DNA topoisomerase II inhibitors: oxolinic acid.

(2) Karyokinesis inhibitor and cell division inhibitors:

(a) β-Tubulin polymerization inhibitors: benomyl, carbendazim, chlorfenazole, fuberidazole, thiabendazole, thiophanate, thiophanate-methyl, diethofencarb, zoxamide, and ethaboxam;
(b) Cell division inhibitors: pencycuron; and
(c) Delocalization inhibitors of spectrin-like protein: fluopicolide.

(3) Respiration inhibitors:

(a) Complex I NADH oxidoreductase inhibitors: diflumetorim and tolfenpyrad;
(b) Complex II succinic acid dehydrogenase inhibitors: benodanil, flutolanil, mepronil, isofetamid, fluopyram; fenfuram, furmecyclox, carboxin, oxycarboxin, thifluzamide, benzovindiflupyr, bixafen, fluxapyroxad, furametpyr, isopyrazam, penflufen, penthiopyrad, sedaxane, boscalid, and pyraziflumid, pydiflumetofen, isoflucypram, and inpyrfluxam;
(c) Complex III ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone; fluoxastrobin, fenamidone, pyribencarb, mandestrobin, and metyltetraprole;
(d) Complex III ubiquinol reductase Qi inhibitors: cyazofamid, amisulbrom, and fenpicoxamid;
(e) Oxidative phosphorylation uncoupling agents: binapacryl, meptyldinocap, dinocap, fluazinam, and ferimzone;
(f) Oxidative phosphorylation inhibitors (ATP synthase inhibitors): fentin acetate, fentin chloride, and fentin hydroxide;
(g) ATP production inhibitors: silthiofam; and
(h) Complex III: cytochrome bcl (ubiquinone reductase) Qx (unknown) inhibitors: ametoctradin.

(4) Amino acid and protein synthesis inhibitors:

(a) Methionine biosynthesis inhibitors: andoprim, cyprodinil, mepanipyrim, and pyrimethanil; and
(b) Protein synthesis inhibitors: blasticidin S; kasugamycin, kasugamycin hydrochloride, streptomycin, and oxytetracycline.

(5) Signal transduction inhibitors:

(a) Signal transduction inhibitors: quinoxyfen and proquinazid; and
(b) MAP/histidine kinase inhibitors in osmotic pressure signal transduction: fenpiclonil, fludioxonil, chlozolinate, iprodione, procymidone, and vinclozolin.

(6) Lipid and cell membrane synthesis inhibitors:

(a) Phospholipid biosynthesis and methyltransferase inhibitors: edifenphos, iprobenfos, pyrazophos, and isoprothiolane;
(b) Lipid peroxidation agents: biphenyl, chloroneb, dichloran, quintozene, tecnazene, tolclofos-methyl, and etridiazole;
(c) Agents that act upon cell membranes: iodocarb, propamocarb, propamocarb-hydrochloride, propamocarb-fosetylate, and prothiocarb;
(d) Microorganisms that disturb pathogen cell membranes: Bacillus subtilis, Bacillus subtilis strain QST713, Bacillus subtilis strain FZB24, Bacillus subtilis strain MBI600, Bacillus subtilis strain D747, and Bacillus amyloliquefaciens; and
(e) Agents that disturb cell membranes: Melaleuca alternifolia (tea tree) extract.

(7) Cell membrane sterol biosynthesis inhibitors:

(a) C14-position demethylation inhibitors in sterol biosynthesis: triforine, pyrifenox, pyrisoxazole, fenarimol, flurprimidol, nuarimol, imazalil, imazalil-sulfate, oxpoconazole, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole, prothioconazole, voriconazole, and mefentrifluconazole;
(b) $\Delta$14 reductase and $\Delta 8 \rightarrow \Delta 7$-isomerase inhibitors in sterol biosynthesis: aldimorph, dodemorph, dodemorph acetate, fenpropimorph, tridemorph, fenpropidin, piperalin, and spiroxamine;
(c) 3-keto reductase inhibitors in C4-position demethylation in sterol biosynthesis systems: fenhexamid and fenpyrazamine; and
(d) Squalene epoxidase inhibitors in sterol biosynthesis systems: pyributicarb, naftifene, and terbinafine.

(8) Cell wall synthesis inhibitors:

(a) Trehalase inhibitors: validamycin;
(b) Chitin synthase inhibitors: polyoxins and polyoxorim; and
(c) Cellulose synthase inhibitors: dimethomorph, flumorph, pyrimorph; benthiavalicarb isopropyl, iprovalicarb, valifenalate, and mandipropamide.

(9) Melanin biosynthesis inhibitors:

(a) Reductase inhibitors in melanin biosynthesis: fthalide, pyroquilon, and tricyclazole;
(b) Anhydrase inhibitors in melanin biosynthesis: carpropamid, diclocymet, and fenoxanil; and
(c) Polyketide inhibitors in melanin biosynthesis: tolprocarb.

(10) Host plant resistance-inducing agents:

(a) Agent that acts on salicylic acid biosynthetic pathway: acibenzolar-S-methyl; and
(b) Others: probenazole; tiadinil; isotianil; dichlobentiazox; laminarin; and Reynoutria sachalinensis extract.

(11) Agents for which the mode of activity is unclear: cymoxanil, fosetyl-aluminum, phosphoric acid (phosphate), tecloftalam, triazoxide, flusulfamide, diclomezine, methasulfocarb, cyflufenamid, metrafenone, pyriofenone, dodine, dodine free base, and flutianil.
(12) Agents having multiple activities: copper (copper salts), bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, iminoctadine triacetate, iminoctadine trialbesilate, anilazine, dithianon, quinomethionate, and fluoroimide.
(13) Other agents: DBEDC, fluor folpet, guazatine acetate, bis(8-quinolinolato) copper (II), propamidine, chloropicrin, cyprofuram, agrobacterium, bethoxazin, diphenylamine, methyl isothiocyanate (MITC), mildiomycin, capsaicin, curfraneb, cyprosulfamide, dazomet, debacarb, dichlorophen, flumetover, fosetyl-calcium, fosetyl-sodium, irumamycin, natamycin, nitrothal isopropyl, oxamocarb, pyrrolnitrin, tebufloquin, tolnifanide, zarilamide, algophase, amicarthiazol, oxathiapiprolin, metiram zinc, benthiazole, trichlamide, uniconazole, oxyfenthiin, picarbutrazox, dichlobentiazox, quinofumelin, thiuram, ambam, agrobacterium radiobacter, coniothyrium minitans, pseudomonas fluorescens,

pseudomonas rhodesiae, talaromyces flavus, trichoderma atroviride, erwinia carotovora subsp. carotovora, bacillus simplex, variovorax paradoxus, lactobacillus plantarum, florylpicoxamid, pyrapropoyne, fluindapyr, aminopyrifen, pyridachlometyl, and ipflufenoquin.

Specific examples of plant growth regulators that can be mixed or used in combination with the formulation for controlling harmful organism of the present invention are listed below.

Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinylglycine (alternative name: aviglycine), aminooxyacetate, silver nitrate, cobalt chloride, IAA, 4-CPA, cloprop, 2,4-D, MCPB, indole 3-butyrate, dichlorprop, phenothiol, 1-naphthyl acetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyl adenine, 5-aminolevulinic acid, and daminozide.

[Formulation for controlling ectoparasites]

The formulation for controlling ectoparasites according to the present invention contains at least one compound selected from the heteroarylpyrimidine compounds of the present invention as an active ingredient. The amount of the heteroarylpyrimidine compounds contained in the formulation for controlling ectoparasites of the present invention is not particularly limited, as long as effects of controlling ectoparasites are exhibited.

As examples of the host animals for which the formulation for controlling ectoparasites of the present invention is applicable, mention may be made of warm-blooded animals such as a pet animal such as a dog or a cat; a pet bird; a farm animal such as cattle, horse, pig, or sheep; domestic fowl; and the like. In addition, honey-bees, stag beetles, unicorn beetles may be mentioned.

The formulation for controlling ectoparasites of the present invention can be applied by a known veterinary method (topical, oral, parenteral or subcutaneous administration). Examples of the method include a method for orally administering tablets, capsules and drinks mixed with the formulation for controlling ectoparasites to the animals; a method for administering to the animals by using an immersion liquid, suppository or injection (intramuscular, subcutaneous, intravenous, intraabdominal or the like); a method for topically administering an oil-based or aqueous liquid preparation by spraying, pouring on, spotting on or the like; a method for topically administering by attaching a collar, an ear tag or the like made by molding a mixture obtained by kneading the formulation for controlling ectoparasites with a resin to the animals; and the like.

The ectoparasites live on the host animals, especially live inside or upon warm-blooded animals. More specifically, the ectoparasites are parasitic in the back, armpit, underbelly, inner thigh and the like of the host animals and obtain nutritional sources such as blood, dandruff from the animals to live.

As examples of ectoparasites, mention may be made of mites, lice, fleas, mosquitoes, stable flies, flesh flies, and the like.

Specific examples of the ectoparasites which can be prevented by the formulation for controlling ectoparasites according to the present invention are described below.

(1) Acari

Acari belonging to the Dermanyssidae family, acari belonging to the Macronyssidae family, acari belonging to the Laelapidae family, acari belonging to the Varroidae family, acari belonging to the Argasidae family, acari belonging to the Ixodidae family, acari belonging to the Psoroptidae family, acari belonging to the Sarcoptidae family, acari belonging to the Knemidokoptidae family, acari belonging to the Demodixidae family, acari belonging to the Trombiculidae family, and insect-parasitic acari such as Coleopterophagus berlesei.

(2) Phthiraptera order

Lice belonging to the Haematopinidae family, lice belonging to the Linognathidae family, biting lice belonging to the Menoponidae family, biting lice belonging to the Philopteridae family, and biting lice belonging to the Trichodectidae family.

(3) Siphonaptera order

Fleas belonging to the Pulicidae family, for example, Ctenocephalides canis and Ctenocephalides felis of Ctenocephalides spp.;

Fleas belonging to the Tungidae family, fleas belonging to the Ceratophyllidae family, and fleas belonging to the Leptopsyllidae family.

(4) Hemiptera order.

(5) Harmful organism of Diptera order

Mosquitoes belonging to the Culicidae family, black flies belonging to the Simuliidae family, punkie belonging to the

Ceratopogonidae family, flies belonging to the Tabanidae family, flies belonging to the Muscidae family, tsetse flies belonging to the Glossinidae family, flesh flies belonging to the Sarcophagidae family, flies belonging to the Hippoboscidae family, flies belonging to the Calliphoridae family, and flies belonging to the Oestridae family;

[Endoparasite Controlling formulation or Parasiticide]

An endoparasite controlling formulation or parasiticide of the present invention contains at least one compound selected from the heteroarylpyrimidine compounds of the present invention as an active ingredient. The amount of the heteroarylpyrimidine compound contained in the endoparasite controlling formulation or parasiticide of the present invention is not particularly limited as long as an endoparasite-controlling effect or a parasiticidal effect is exhibited.

The parasites targeted by the endoparasite controlling formulation or parasiticide of the present invention live in host animals, and particularly in warm-blooded animals and fish (namely, endoparasites). Examples of host animals for which the endoparasite controlling formulation or parasiticide of the present invention is effective include warm-blooded animals such as humans, domestic mammals (for example, cows, horses, pigs, sheep, and goats and the like), experimental animals (for example, mice, rats, and gerbils and the like), pet animals (for example, hamsters, guinea pigs, dogs, cats, horses, squirrels, rabbits, and ferrets and the like), wild mammals and zoo mammals (for example, monkeys, foxes, deer, and buffalo and the like), domestic fowl (for example, turkeys, ducks, chickens, quail, and geese, and the like), and pet birds (for example, pigeons, parrots, myna birds, Java finches, parakeets, Bengalese finches, and canaries and the like); and fishes such as salmon, trout, and koi carp and the like. By controlling or exterminating the parasites, parasitic diseases carried by the parasites can be prevented or treated.

Examples of parasites that can be controlled or exterminated include those listed below.

(1) Dioctophymatida nematodes

(a) Kidney worms of the Dioctophymatidae family, for example, Dioctophyma renale of Dioctophyma spp.; and
(b) Kidney worms of the Soboliphymatidae family, for example, Soboliphyme abei and Soboliphyme baturini of Soboliphyme spp.

(2) Trichocephalida nematodes

(a) Trichina worms of the Trichinellidae family, for example, Trichinella spiralis of Trichinella spp.; and
(b) Whipworms of the Trichuridae family, for example, Capillaria annulata, Capillaria contorta, Capillaria hepatica, Capillaria perforans, Capillaria plica and Capillaria suis of Capillaria spp.; and Trichuris vulpis, Trichuris discolor, Trichuris ovis, Trichuris skrjabini and Trichuris suis of Trichuris spp.

(3) Rhabditida nematodes
Threadworms of the Strongyloididae family, for example, Strongyloides papillosus, Strongyloides planiceps, Strongyloides ransomi, Strongyloides suis, Strongyloides stercoralis, Strongyloides tumefaciens and Strongyloides ratti of Strongyloides spp.
(4) Strongylida nematodes
Hookworms of the Ancylostomatidae family, for example, Ancylostoma braziliense, Ancylostoma caninum, Ancylostoma duodenale and Ancylostoma tubaeforme of Ancylostoma spp.; Uncinaria stenocephala of Uncinaria stenocephala; and Bunostomum phlebotomum and Bunostomum trigonocephalum of Bunostomum spp.
(5) Strongylida nematodes

(a) Nematodes of the Angiostrongylidae family, for example, Aelurostrongylus abstrusus of Aelurostrongylus spp.; and Angiostrongylus vasorum and Angiostrongylus cantonesis of Angiostrongylus spp.;
(b) Nematodes of the Crenosomatidae family, for example, Crenosoma aerophila and Crenosoma vulpis of Crenosoma spp.;
(c) Nematodes of the Filaroididae family, for example, Filaroides hirthi and Filaroides osleri of Filaroides spp.;
(d) Lungworms of the Metastrongylidae family, for example, Metastrongylus apri, Metastrongylus asymmetricus, Metastrongylus pudendotectus and Metastrongylus salmi of Metastrongylus spp.; and
(e) Gapeworms of the Syngamidae family, for example, Cyathostoma bronchialis of Cyathostoma spp.; and Syngamus skrjabinomorpha and Syngamus trachea of Syngamus spp.

(6) Strongylida nematodes

(a) Nematodes of the Molineidae family, for example, Nematodirus filicollis and Nematodirus spathiger of Nematodirus spp.;
(b) Nematodes of the Dictyocaulidae family, for example, Dictyocaulus filarial and Dictyocaulus viviparus of Dictyocaulus spp.;
(c) Nematodes of the Haemonchidae family, for example, Haemonchus contortus of Haemonchus spp.; and Mecistocirrus digitatus of Mecistocirrus spp.;
(d) Nematodes of the Haemonchidae family, for example, Ostertagia ostertagi of Ostertagia spp.;
(e) Nematodes of the Heligmonellidae family, for example, Nippostrongylus braziliensis of Nippostrongylus spp.; and
(f) Nematodes of the Trichostrongylidae family, for example, Trichostrongylus axei, Trichostrongylus colubriformis and Trichostrongylus tenuis of Trichostrongylus spp.; Hyostrongylus rubidus of Hyostrongylus spp.; and Obeliscoides cuniculi of Obeliscoides spp.

(7) Strongylida nematodes

(a) Nematodes of the Chabertiidae family, for example, Chabertia ovina of Chabertia spp.; and Oesophagostomum brevicaudatum, Oesophagostomum columbianum, Oesophagostomum dentatum, Oesophagostomum georgianum, Oesophagostomum maplestonei, Oesophagostomum quadrispinulatum, Oesophagostomum radiatum, Oesophagostomum venulosum and Oesophagostomum watanabei of Oesophagostomum spp.;
(b) Nematodes of the Stephanuridae family, for example, Stephanurus dentatus of Stephanurus spp.; and
(c) Nematodes of the Strongylidae family, for example, Strongylus asini, Strongylus edentatus, Strongylus equinus and Strongylus vulgaris of Strongylus spp.

(8) Oxyurida nematodes
Nematodes of the Oxyuridae family, for example, Enterobius anthropopitheci and Enterobius vermicularis of Enterobius spp.; Oxyuris equi of Oxyuris spp.; and Passalurus ambiguus of Passalurus spp.
(9) Ascaridida nemtaodes

(a) Nematodes of the Ascaridiidae family, for example, Ascaridia galli of Ascaridia spp.;
(b) Nematodes of the Heterakidae family, for example, Heterakis beramporia, Heterakis brevispiculum, Heterakis gallinarum, Heterakis pusilla and Heterakis putaustralis of Heterakis spp.;
(c) Nematodes of the Anisakidae family, for example, Anisakis simplex of Anisakis spp.;
(d) Nematodes of the Ascarididae family, for example, Ascaris lumbricoides and Ascaris suum of Ascaris spp.; and Parascaris equorum of Parascaris spp.; and
(e) Nematodes of the Toxocaridae family, for example, Toxocara canis, Toxocara leonina, Toxocarasuum, Toxocara vitulorum and Toxocara cati of Toxocara spp.

(10) Spirurida nematodes

(a) Nematodes of the Onchocercidae family, for example, Brugia malayi, Brugia pahangi and Brugia patei of Brugia spp.; Dipetalonema reconditum of Dipetalonema spp.; Dirofilaria immitis of Dirofilaria spp.; Filaria oculi of Filaria spp.; and Onchocerca cervicalis, Onchocerca gibsoni and Onchocerca gutturosa of Onchocerca spp.
(b) Nematodes of the Setariidae family, for example, Setaria digitata, Setaria equina, Setaria labiatopapillosa and Setaria marshalli of Setaria spp.; and Wuchereria bancrofti of Wuchereria spp.; and
(c) Nematodes of the Filariidae family, for example, Parafilaria multipapillosa of Parafilaria spp.; and Stephanofilaria assamensis, Stephanofilaria dedoesi, Stephanofilaria kaeli, Stephanofilaria okinawaensis and Stephanofilaria stilesi of Stephanofilaria spp.

(11) Spirurida nematodes

(a) Nematodes of the Gnathostomatidae family, for example, Gnathostoma doloresi and Gnathostoma spinigerum of Gnathostoma spp.;
(b) Nematodes of the Habronematidae family, for example, Habronema majus, Habronema microstoma and Habronema muscae of Habronema spp.; and Draschia megastoma of Draschia spp.;
(c) Nematodes of the Physalopteridae family, for example, Physaloptera canis, Physaloptera cesticillata, Physaloptera erdocyona, Physaloptera felidis, Physaloptera gemina, Physaloptera papilloradiata, Physaloptera praeputialis, Physaloptera pseudopraerutialis, Physaloptera rara, Physaloptera sibirica and Physaloptera vulpineus of Physaloptera spp.;
(d) Nematodes of the Gongylonematidae family, for example, Gongylonema pulchrum of Gongylonema spp.;

(e) Nematodes of the Spirocercidae family, for example, Ascarops strongylina of Ascarops spp.; and
(f) Nematodes of the Thelaziidae family, for example, Thelazia callipaeda, Thelazia gulosa, Thelazia lacrymalis, Thelazia rhodesi and Thelazia skrjabini of Thelazia spp.

[Formulation for Controlling Other Harmful Organisms]

In addition, the heteroarylpyrimidine compound of the present invention exhibits a superior effect for controlling other pests that have a sting or venom that can harm humans and animals, pests carrying various pathogens / pathogenic bacteria, and pests that impart a discomforting sensation to humans (such as toxic pests, sanitary insect pests, unpleasant insect pests).
Specific examples thereof are listed below.

(1) Hymenoptera insect pests

Sawflies of the Argidae family, wasps of the Cynipidae family, sawflies of the Diprionidae family, ants of the Formicidae family, wasps of the Mutillidae family, and wasps of the Vespidae family.

(2) Other insect pests

Blattodea, termites, Araneae, centipedes, millipedes, crustacea and Cimex lectularius.

EXAMPLES

[Formulation Examples]

Some examples of the formulations for controlling harmful organisms, insecticides, acaricides, formulations for controlling ectoparasites, or formulations for controlling or expelling endoparasites of the present invention are described below. The additives and the addition ratios are not limited to those in the examples and can be modified over a wide range. The term "part" in the formulation examples indicates "part by weight".
The formulation examples for agricultural and horticultural use and for paddy rice are described below.

(Formulation 1: Wettable powder)

40 parts of the compound of the present invention, 53 parts of diatomaceous earth, 4 parts of a higher alcohol sulfuric ester, and 3 parts of an alkylnaphthalene sulfonic acid salt were uniformly mixed and finely pulverized to obtain a wettable powder including 40% of an active ingredient.

(Formulation 2: Emulsion)

30 parts of the compound of the present invention, 33 parts of xylene, 30 parts of dimethylformamide and 7 parts of a polyoxyethylene alkyl aryl ether were mixed and dissolved to obtain an emulsion including 30% of an active ingredient.

(Formulation 3: Granules)

5 parts of the compound of the present invention, 40 parts of talc, 38 parts of clay, 10 parts of bentonite and 7 parts of sodium alkylsulfate were uniformly mixed and finely pulverized, followed by granulating into a granular shape having a diameter of 0.5 to 1.0 mm to obtain granules containing 5% of an active ingredient.

(Formulation 4: Granules)

5 parts of the compound of the present invention, 73 parts of clay, 20 parts of bentonite, 1 part of sodium dioctyl sulfosuccinate and 1 part of potassium phosphate were thoroughly pulverized and mixed. Water was added thereto, and the mixture was kneaded well, followed by granulating and drying to obtain granules containing 5% of an active ingredient.

(Formulation 5: Suspension)

10 parts of the compound according to the present invention, 4 parts of polyoxyethylene alkyl allyl ether, 2 parts of sodium polycarboxylate, 10 parts of glycerol, 0.2 parts of xanthan gum and 73.8 parts of water were mixed and wet-pulverized so as to have a grain size of 3 microns or less. Thereby, a suspension containing 10% of an active ingredient was obtained.

The formulation examples of the formulation for controlling ectoparasites, or the formulation for controlling or expelling endoparasites are described below.

(Formulation 6: Granulated powder)

5 parts of the compound of the present invention was dissolved in an organic solvent to obtain a solution. The solution mentioned above was sprayed on 94 parts of kaolin and 1 part of white carbon, followed by evaporating the solvent under reduced pressure. This type of granulated powder may be mixed with animal food.

(Formulation 7: Impregnating formulation)

0.1 to 1 parts of the compound of the present invention and 99 to 99.9 parts of peanut oil were uniformly mixed, and then filter-sterilized by means of a sterilizing filter.

(Formulation 8: Pour-on formulation)

5 parts of the compound of the present invention, 10 parts of a myristic ester and 85 parts of isopropanol were uniformly mixed to obtain a pour-on formulation.

(Formulation 9: Spot-on formulation)

10 to 15 parts of the compound of the present invention, 10 parts of a palmitic ester and 75 to 80 parts of isopropanol were uniformly mixed to obtain a spot-on formulation.

(Formulation 10: Spray formulation)

1 part of the compound of the present invention, 10 parts of propylene glycol and 89 parts of isopropanol were uniformly mixed to obtain a spray formulation.

Next, Examples of compounds are described to explain the present invention more specifically. It should be understood that the present invention is not limited to the following examples of compounds.

[Example 1]

Synthesis of 2-(5-(ethylsulfonyl)-[2,2'-bipyrimidin]-4-yl)-3-methyl-6-(trifluoromethyl)-3H-imidazo[4,5-b]pyridine (Compound No. A-2)

(Step 1)

Synthesis of 3-methyl-6-(trifluoromethyl)-3H-imidazo[4,5-b]pyridine

[Chem. 5]

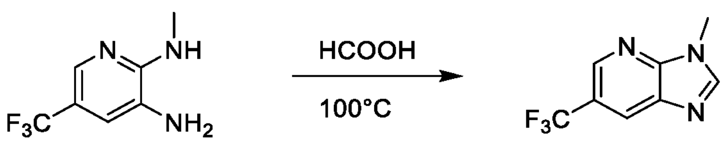

Formic acid (16 ml) was added to $N^2$-methyl-5-(trifluoromethyl) pyridin-2,3-diamine (2 g) synthesized by means of the method described in WO 2012/086848 A. The mixture was stirred for 2 hours at 100°C. The resultant reaction solution was poured into a saturated aqueous solution of sodium hydrogen carbonate, and the mixture was subjected to extraction with ethyl acetate. The obtained organic layer was washed with a saturated aqueous solution of sodium chloride, dried over anhydrous magnesium sulfate, and filtered. The filtrate was concentrated under reduced pressure. The obtained residue was suspended in normal-hexane, and filtered. Thereby, the objective product was obtained in an amount of 1.2 g (yield 57%).

$^1$H-NMR of the objective product obtained is shown below.

$^1$H-NMR (400 MHz, DMSO-$d_6$): δ 8.77 (d, 1H), 8.67 (s, 1H), 8.53 (d, 1H), 3.91 (s, 3H).

(Step 2)

Synthesis of 1-(3-methyl-6-(trifluoromethyl)-3H-imidazo[4,5-b] pyridin-2-yl)ethan-1-one

[Chem. 6]

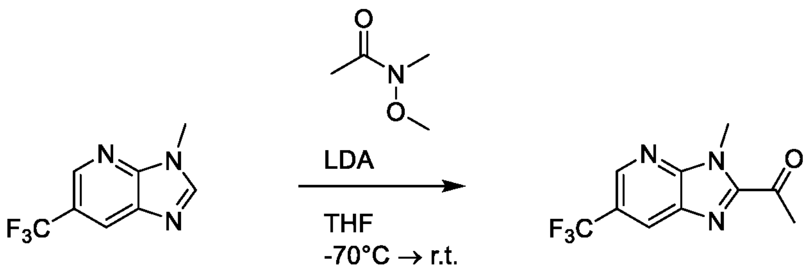


3-Methyl-6-(trifluoromethyl)-3H-imidazo[4,5-b]pyridine (2.8 g) was dissolved in tetrahydrofuran (100 ml), and the reaction container was subjected to substitution with nitrogen. Subsequently, the solution was stirred at -70°C. Lithium diisopropylamide (40 ml, ca. 0.38 M tetrahydrofuran solution) was added dropwise thereto, and the mixture was stirred for 30 minutes at -70°C. N-methoxy-N-methylacetamide (2.2 g) was added dropwise thereto. The mixture was stirred for one hour at -70°C, and subsequently, the mixture was warmed to room temperature. The resultant reaction solution was poured into a saturated aqueous solution of ammonium chloride, and the mixture was subjected to extraction with ethyl acetate. The obtained organic layer was washed with a saturated aqueous solution of sodium chloride, dried over anhydrous magnesium sulfate, and filtered. The filtrate was concentrated under reduced pressure. The obtained residue was purified with column chromatography with silica gel. Thereby, the objective product was obtained in an amount of 2.4 g (yield 70%).

$^1$H-NMR of the objective product obtained is shown below.

$^1$H-NMR (400 MHz, $CDCl_3$): δ 8.83 (d, 1H), 8.44 (d, 1H), 4.24 (s, 3H), 2.86 (s, 3H).

(Step 3)

Synthesis of 2-bromo-1-(3-methyl-6-(trifluoromethyl)-3H-imidazo [4,5-b]pyridin-2-yl)ethan-1-one

[Chem. 7]

20

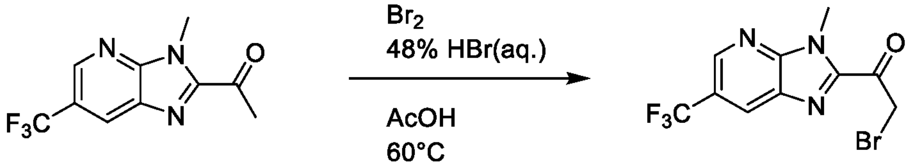


1-(3-Methyl-6-(trifluoromethyl)-3H-imidazo[4,5-b]pyridin-2-yl)ethan-1-one (2.7g) was dissolved in acetic acid (50 ml), and stirred at room temperature. Bromine (1.8 g) and 48% hydrobromic acid (2 ml) were added thereto. The mixture was stirred for one hour at 60°C. The resultant reaction solution was concentrated under reduced pressure. The residue was poured into a saturated solution of sodium hydrogen carbonate, and the mixture was subjected to extraction with dichloromethane. The obtained organic layer was washed with a saturated aqueous solution of sodium chloride, dried over anhydrous magnesium sulfate, and filtered. The filtrate was concentrated under reduced pressure. The obtained residue was used in the next step, without purification.

$^1$H-NMR of the objective product obtained is shown below.

$^1$H-NMR (400 MHz, $CDCl_3$): $\delta$ 8.86 (d, 1H), 8.47 (d, 1H), 4.85 (s, 2H), 4.26 (s, 3H).

(Step 4)

Sythesis of 2-(ethylthio)-1-(3-methyl-6-(trifluoromethyl)-3H-imidazo[4,5-b] pyridin-2-yl)ethan-1-one

[Chem. 8]

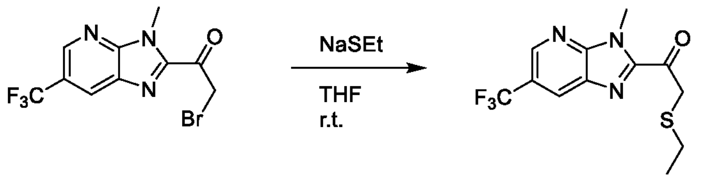


2-Bromo-1-(3-methyl-6-(trifluoromethyl)-3H-imidazo [4,5-b]pyridin-2-yl)ethan-1-one obtained in step 3 was dissolved in tetrahydrofuran (110 ml), and the mixture was stirred at 0°C. Ethyl mercaptan sodium salt (0.64 g, 80%) was added thereto, and the mixture was stirred for one hour at room temperature. The resultant liquid was poured into water. The mixture was subjected to extraction with ethyl acetate. The reaction solution mentioned above was washed with a saturated aqueous solution of sodium chloride, dried over anhydrous magnesium sulfate, and filtered. The filtrate was concentrated under reduced pressure. The obtained residue was used in the next step, without purification.

$^1$H-NMR of the objective product obtained is shown below.

$^1$H-NMR (400 MHz, $CDCl_3$): $\delta$ 8.82 (d, 1H), 8.45 (d, 1H), 4.25 (s, 3H), 4.12 (q, 2H), 4.07 (s, 2H), 1.31 (t, 3H).

(Step 5)

Synthesis of 2-(ethylsulfonyl)-1-(3-methyl-6-(trifluoromethyl)-3H-imidazo [4,5-b]pyridin-2-yl)ethan-1-one

[Chem. 9]

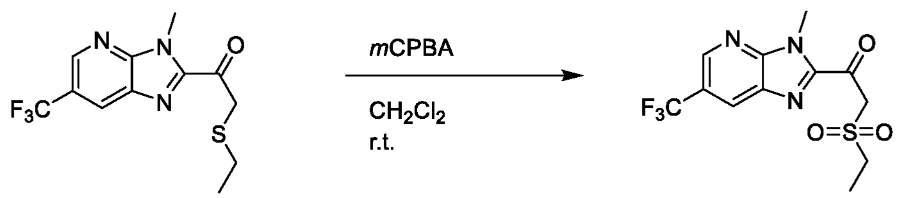


2-(Ethylthio)-1-(3-methyl-6-(trifluoromethyl)-3H-imidazo[4,5-b] pyridin-2-yl)ethan-1-one obtained in step 4 was dissolved in dichloromethane (110 ml), and the mixture was stirred at 0°C. Metachloroperbenzoic acid (70%, 5.7 g) was added thereto, and the mixture was stirred for one hour at room temperature. The resultant reaction solution was poured into a mixed solution of a saturated aqueous solution of sodium hydrogen carbonate and a saturated aqueous solution of sodium thiosulfate.

The mixture was subjected to extraction with dichloromethane. The obtained organic layer was washed with a saturated aqueous solution of sodium chloride, dried over anhydrous magnesium sulfate, and filtered. The filtrate was concentrated under reduced pressure, and the obtained residue was purified by column chromatography with silica gel. Thereby, the objective product was obtained in an amount of 1.1 g (yield 28%, 3 steps).

$^1$H-NMR of the objective product obtained is shown below.

$^1$H-NMR (400 MHz, $CDCl_3$): $\delta$ 8.88 (d, 1H), 8.50 (d, 1H), 5.02 (s, 1H), 4.26 (s, 3H), 3.35 (q, 2H), 1.51 (t, 3H).

(Step 6)

Sythesis of 3-(dimethylamino)-2-(ethylsulfonyl)-1-(3-methyl-6-(trifluoromethyl)-3H-imidazo[4,5-b]pyridin-2-yl)prop-2-en-1-one

[Chem. 10]

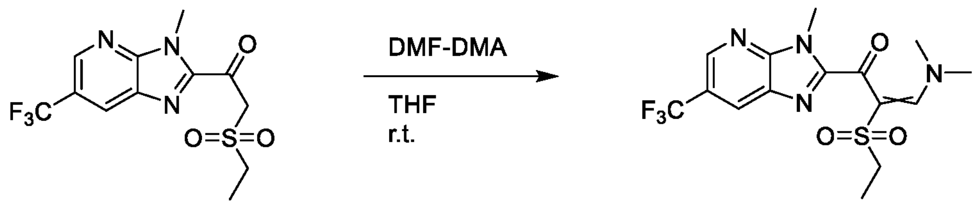


2-(Ethylsulfonyl)-1-(3-methyl-6-(trifluoromethyl)-3H-imidazo [4,5-b]pyridin-2-yl)ethan-1-one (0.38 g) was dissolved in tetrahydrofuran (6 ml), and the mixture was stirred at room temperature. N,N-dimethylformamide dimethylacetal (0.27 g) was added thereto, and the mixture was stirred for one hour at 60°C. The reaction solution was concentrated under reduced pressure. The obtained residue was used in the next step, without purification.

(Step 7)

Synthesis of 2-(5-(ethylsulfonyl)-[2,2'-bipyrimidin]-4-yl)-3-methyl-6-(trifluoromethyl)-3H-imidazo[4,5-b]pyridine

[Chem. 11]

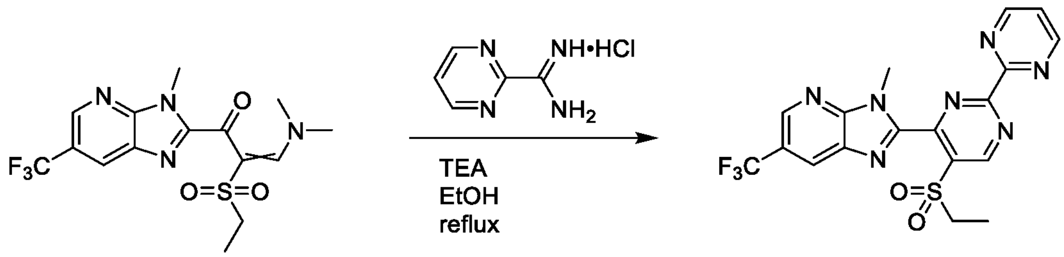


3-(Dimethylamino)-2-(ethylsulfonyl)-1-(3-methyl-6-(trifluoromethyl)-3H-imidazo[4,5-b]pyridin-2-yl)prop-2-en-1-one obtained in step 6 was dissolved in ethanol (6 ml), and the mixture was stirred at room temperature. Triethylamine (0.33 g) and 2-amidinopyrimidine hydrochloride (0.26 g) were added thereto, and the mixture was stirred for one hour under heating and refluxing. The resultant solution was poured into water, and the mixture was subjected to extraction with chloroform. The reaction solution was washed with a saturated aqueous solution of sodium chloride, dried over anhydrous magnesium sulfate, and filtered. The filtrate was concentrated under reduced pressure, and the obtained residue was purified by column chromatography with silica gel. Thereby, the objective product was obtained in an amount of 0.16 g (yield 33%, 2 steps).

$^1$H-NMR of the objective product obtained is shown below.

$^1$H-NMR (400 MHz, $CDCl_3$): δ 9.73 (s, 1H), 9.10 (d, 1H), 8.82 (d, 1H), 8.36 (d, 1H), 7.56 (t, 1H), 4.12 (s, 3H), 4.05 (q, 2H), 1.48 (t, 3H).

[Example 2]

Synthesis of 2-(5-(ethylsulfonyl)-[2,2'-bipyrimidin]-4-yl)-5-((trifluoromethyl)thio)benzo[d]oxazole (Compound No. A-7)

(Step 1)

Synthesis of methyl 5-((trifluoromethyl)thio)benzo[d]oxazole-2-carboxylate

[Chem. 12]

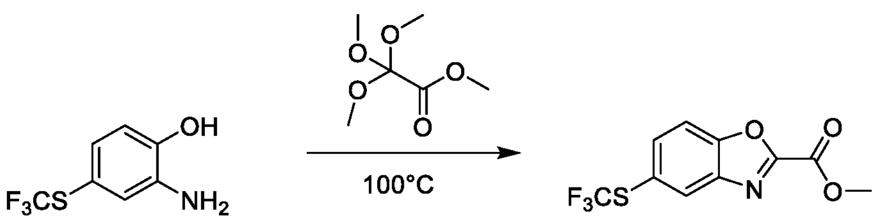


Methyl trimethoxyacetate (3.0 g) was added to 2-amino-4-((trifluoromethyl) thio)) phenol (3.8 g) synthesized in accordance with the method described in WO 2011/049222 A, and the mixture was stirred overnight at 100°C. The resultant reaction solution was concentrated under reduced pressure, and the obtained residue was purified by column chromatography with silica gel. Thereby, the objective product was obtained in an amount of 3.9 g (yield 77%).

$^1$H-NMR of the objective product obtained is shown below.

$^1$H-NMR (400 MHz, $CDCl_3$): δ 8.23 (s, 1H), 7.83 (d, 1H), 7.73 (d, 1H), 4.12 (s, 3H).

(Step 2)

Synthesis of N-methoxy-N-methyl-5-((trifluoromethyl)thio)benzo [d]oxazole-2-carboxamide

[Chem. 13]

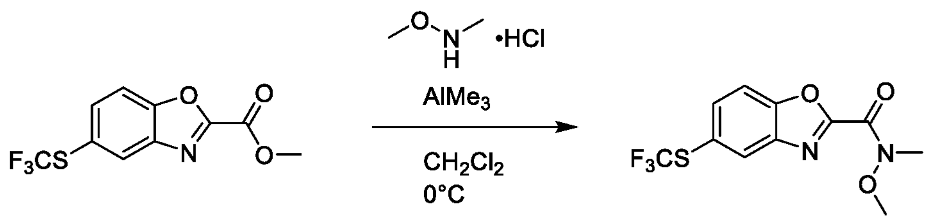


N,O-dimethylhydroxyamine hydrochloride (6.8 g) was suspended in dichloromethane (215 ml), and the reaction container was subjected to substitution with nitrogen. Subsequently, the mixture was stirred at 0°C. Trimethylaluminum (39 ml, about 1.8 M normal-hexane solution) was added dropwise thereto, and the mixture was stirred for 30 minutes at 0°C. A solution of methyl 5-((trifluoromethyl)thio)benzo[d] oxazole-2-carboxylate (3.9 g) dissolved in dichloromethane (215 ml) was added dropwise thereto, and the mixture was stirred for one hour at 0°C. The resultant reaction solution was poured into a saturated aqueous solution of tripotassium phosphate, and the mixture was subjected to extraction with dichloromethane. The obtained organic layer was washed with a saturated aqueous solution of sodium chloride, dried over anhydrous magnesium sulfate, and filtered. The filtrate was concentrated under reduced pressure, and the obtained residue was purified by column chromatography with silica gel. Thereby, the objective product was obtained in an amount of 2.6 g (yield 60%).

$^1$H-NMR of the objective product obtained is shown below.

$^1$H-NMR (400 MHz, $CDCl_3$): δ 8.19 (s, 1H), 7.78 (d, 1H), 7.70 (d, 1H), 3.94 (s, 3H), 3.44 (br s, 3H).

(Step 3)

Synthesis of 2-(ethylsulfonyl)-1-(5-((trifluoromethyl)thio)benzo [d]oxazol-2-yl)ethan-1-one

[Chem. 14]

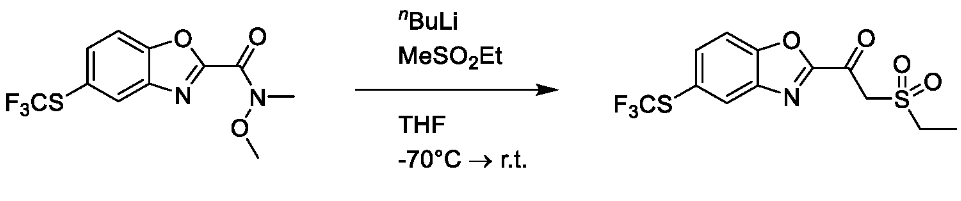


The inside of the reaction container was substituted with nitrogen, and subsequently, a solution of ethyl methylsulfone (1.2 g) dissolved in tetrahydrofuran (8 ml) was added dropwise to a mixture of tetrahydrofuran (8 ml) and n-butyl lithium (3.2 ml, 2.65 M normal-hexane solution) at -70°C. The mixture was stirred for 10 minutes at 0°C. The mixture was cooled to -70°C, and

N-methoxy-N-methyl-5-((trifluoromethyl)thio)benzo [d]oxazole-2-carboxamide (2.6 g) was added thereto. The mixture was warmed to room temperature. The resultant reaction solution was poured into a saturated aqueous solution of ammonium chloride, and the mixture was subjected to extraction with ethyl acetate. The obtained organic layer was washed with a saturated aqueous solution of sodium chloride, dried over anhydrous magnesium sulfate, and filtered.

The filtrate was concentrated under reduced pressure, and the obtained residue was purified by column chromatography with silica gel. Thereby, the objective product was obtained in an amount of 2.5 g (yield 83%).

$^1$H-NMR of the objective product obtained is shown below.

$^1$H-NMR (400 MHz, $CDCl_3$): $\delta$ 8.30 (s, 1H), 7.88 (d, 1H), 7.75 (d, 1H), 4.90 (s, 2H), 3.34 (q, 2H), 1.51 (t, 3H).

(Step 4)

Sythesis of 2-(5-(ethylsulfonyl)-[2,2'-bipyrimidin]-4-yl)-5-((trifluoromethyl) thio)benzo[d]oxazole

[Chem. 15]

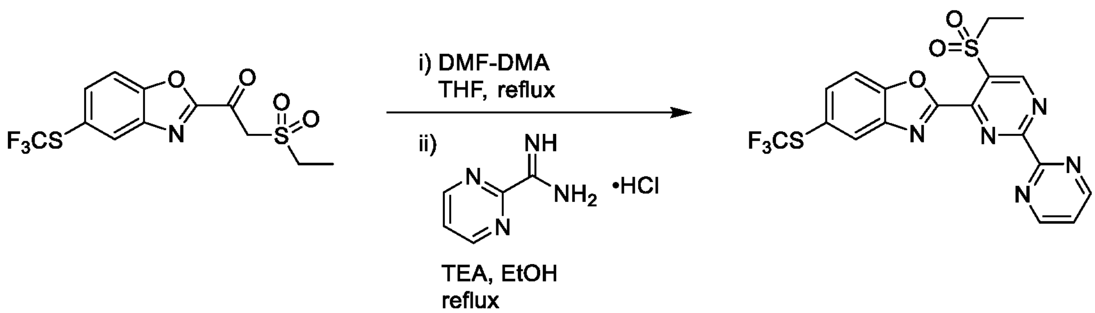


2-(Ethylsulfonyl)-1-(5-((trifluoromethyl)thio)benzo[d]oxazol-2-yl)ethan-1-one (0.47 g) was dissolved in tetrahydrofuran (7 ml), and the mixture was stirred at room temperature. N,N-dimethylformamide dimethyl acetal (0.79 g) was added thereto, and the mixture was stirred for one hour under heating and refluxing. The resulting reaction solution was concentrated under reduced pressure. The obtained residue was dissolved in ethanol (7 ml), and stirred at room temperature. Triethylamine (0.83 ml) and 2-amidinopyrimidine hydrochloride (0.32 g) were added thereto, and the mixture was stirred for one hour under heating and refluxing. The resultant reaction solution was poured into water, and the mixture was subjected to extraction with ethyl acetate. The obtained organic layer was washed with a saturated aqueous solution of sodium chloride, dried over anhydrous magnesium sulfate, and filtered. The filtrate was concentrated under reduced pressure, and the obtained residue was purified by column chromatography with silica gel. Thereby, the objective product was obtained in an amount of 0.26 g (yield 42%).

$^1$H-NMR of the objective product obtained is shown below.

$^1$H-NMR (400 MHz, $CDCl_3$): $\delta$ 9.78 (s, 1H), 9.13 (d, 2H), 8.24 (s, 1H), 7.85 - 7.79 (m, 2H), 7.57 (t, 1H), 4.15 (q, 2H), 1.51 (t, 3H).

[Example 3]

Synthesis of 2-(5-(ethylsulfonyl)-[2,2'-bipyrimidin]-4-yl)-5-((trifluoromethyl) sulfinyl)benzo[d]oxazole (Compound No. A-8)

[Chem. 16]

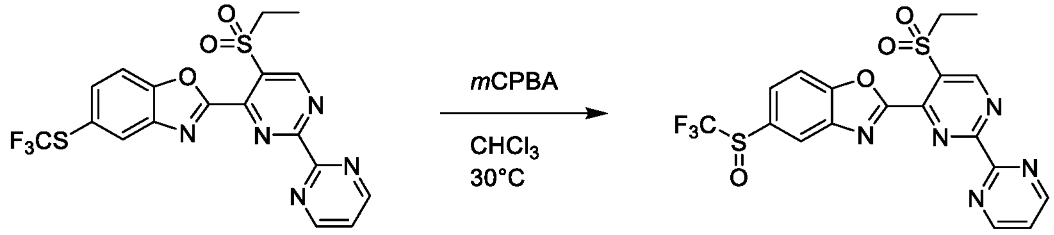


2-(5-(Ethylsulfonyl)-[2,2'-bipyrimidin]-4-yl)-5-((trifluoromethyl) thio)benzo [d]oxazole (0.15 g) was dissolved in chloroform (4 ml), and the mixture was stirred at 0°C. Metachloroperbenzoic acid (70%, 0.17 g) was added thereto, and the mixture was stirred overnight at 30°C. The resultant reaction solution was poured into a mixed solution of a saturated aqueous solution of sodium hydrogen carbonate and a saturated aqueous solution of sodium thiosulfate. The mixture was subjected to extraction with dichloromethane. The obtained organic layer was washed with a saturated aqueous solution of sodium chloride, dried over anhydrous magnesium sulfate, and filtered. The filtrate was concentrated under reduced pressure, and the obtained residue was purified by column chromatography with silica gel. Thereby, the objective product was obtained in an amount of 0.10 g (yield 65%).

$^1$H-NMR of the objective product obtained is shown below.

$^1$H-NMR (400 MHz, $CDCl_3$): δ 9.79 (s, 1H), 9.14 (d, 2H), 8.41 (s, 1H), 7.99 (d, 1H), 7.94 (d, 1H), 7.58 (t, 1H), 4.13 (q, 2H), 1.52 (t, 3H).

[Example 4]

Synthesis of 2-(5-(ethylsulfonyl)-[2,2'-bipyrimidin]-4-yl)-5-((trifluoromethyl) sulfonyl)benzo[d]oxazole (Compound No. A-9)

[Chem. 17]

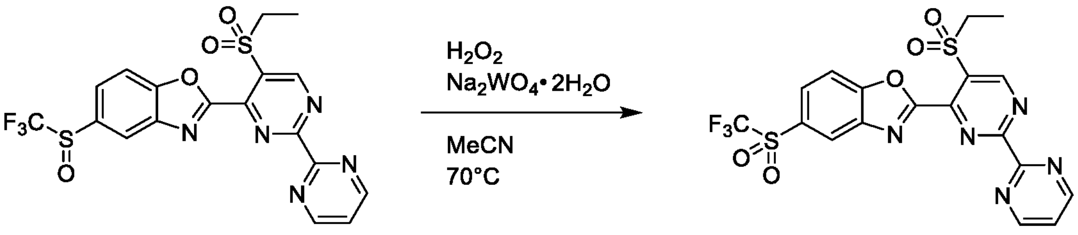


2-(5-(Ethylsulfonyl)-[2,2'-bipyrimidin]-4-yl)-5-((trifluoromethyl) sulfinyl)benzo[d]oxazole (58 mg) was dissolved in acetonitrile (2 ml), and the mixture was stirred at room temperature. Sodium tungstate dihydrate (4 mg) and hydrogen peroxide (30%, 0.5 ml) were added thereto, and the mixture was stirred for 4 hours at 70°C. The resultant reaction solution was poured into a mixed solution of a saturated aqueous solution of sodium hydrogen carbonate and a saturated aqueous solution of sodium thiosulfate. The mixture was subjected to extraction with dichloromethane. The obtained organic layer was washed with a saturated aqueous solution of sodium chloride, dried over anhydrous magnesium sulfate, and filtered. The filtrate was concentrated under reduced pressure, and the obtained residue was purified by column chromatography with silica gel. Thereby, the objective product was obtained in an amount of 15 mg (yield 25%).

$^1$H-NMR of the objective product obtained is shown below.

$^1$H-NMR (400 MHz, $CDCl_3$): δ 9.80 (s, 1H), 9.14 (d, 2H), 8.64 (s, 1H), 8.23 (d, 1H), 8.03 (d, 1H), 7.59 (t, 1H), 4.09 (q, 2H), 1.53 (t, 3H).

[Example 5]

Synthesis of 1-(5-(ethylsulfonyl)-4-(3-methyl-6-(trifluoromethyl)-3*H*-imidazo[4,5-*b*]pyridin-2-yl)pyrimidin-2-yl)-1*H*-1,2,4-triazol-5-amine (Compound No. A-12)

(Step 1)

Synthesis of 2-(2-chloro-5-fluoropyrimidin-4-yl)-3-methyl-6-(trifluoromethyl)-3*H*-imidazo[4,5-*b*]pyridine

[Chem. 18]

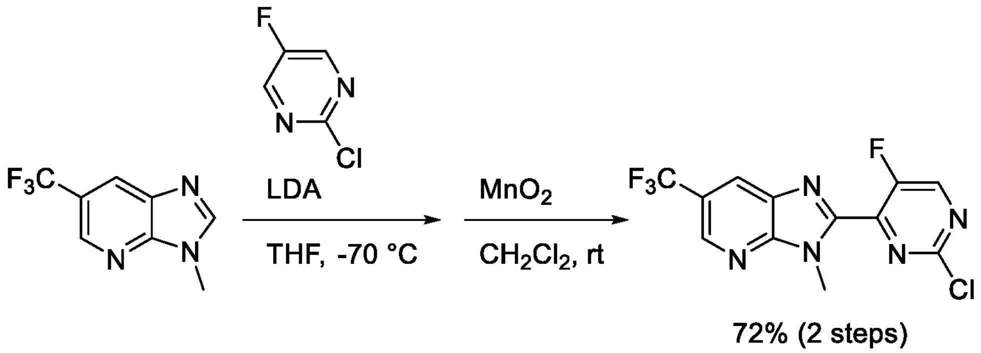


Diisopropylamine (9.1 g) was dissolved in THF (225 ml), and the mixture was stirred at -70°C. *n*-BuLi (2.76 M, 31 ml) was added thereto, and the mixture was stirred for 30 minutes at -70°C. A solution of 3-methyl-6-(trifluoromethyl)-3*H*-imidazo [4,5-*b*]pyridine (15.0 g) was added dropwise thereto, and the mixture was stirred for 30 minutes at -70°C. A solution of 2-chloro-5-fluoropyrimidine (12.9 g) dissolved in THF (26 ml) was added dropwise thereto, and the mixture was stirred for 2 hours at -70°C.

A saturated aqueous solution of ammonium chloride was poured into the resultant liquid, and the mixture was subjected to extraction with ethyl acetate. The obtained organic layer was washed with a saturated aqueous solution of sodium chloride, dried over anhydrous magnesium sulfate, and filtered. The filtrate was concentrated under reduced pressure, and the obtained residue was dissolved in dichloromethane (225 ml). Manganese dioxide (32.5 g) was added thereto, and the mixture was stirred overnight at room temperature. The obtained liquid was filtered with celite, the filtrate was concentrated under reduced pressure. The obtained residue was purified by column chromatography with silica gel. Thereby, the objective product was obtained in an amount of 15 mg (yield 25%).

$^1$H-NMR of the objective product obtained is shown below.

$^1$H-NMR (400 MHz, $CDCl_3$): δ 8.83 (d, 1H), 8.78 (d, 1H), 8.47 (d, 1H), 4.33 (s, 3H).

(Step 2)

Synthesis of 2-(2-chloro-5-(ethylthio)pyrimidin-4-yl)-3-methyl-6-(trifluoromethyl)-3*H*-imidazo[4,5-*b*] pyridine

[Chem. 19]

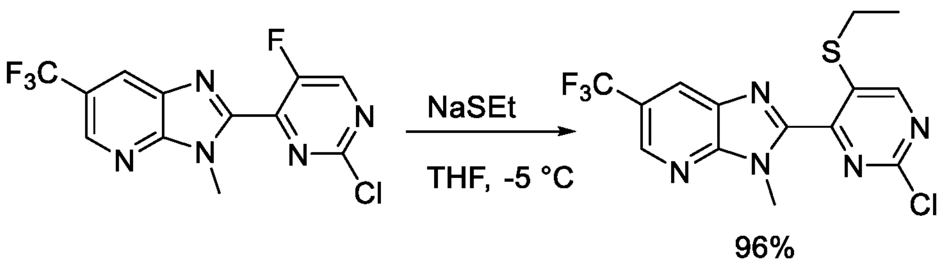


2-(2-chloro-5-fluoropyrimidin-4-yl)-3-methyl-6-(trifluoromethyl)-3*H*-imidazo [4,5-*b*]pyridine (21.3 g) was dissolved in THF (650 ml), and the mixture was stirred at -5°C. Ethyl mercaptan sodium salt (80%, 6.38 g) was added thereto, and the mixture was stirred for 2 hours at -5°C. The resultant liquid was poured into water, and filtered. The obtained crystals were subjected to azeotropic dehydration. Thereby, the objective product was obtained in an amount of 23 g (yield 96%).

$^1$H-NMR of the objective product obtained is shown below.

$^1$H-NMR (400 MHz, $CDCl_3$): δ 8.80 (d, 1H), 8.69 (s, 1H), 8.47 (d, 1H), 4.27 (s, 3H), 3.09 (q, 2H), 1.43 (t, 3H).

(Step 3)

Synthesis of 2-(2-chloro-5-(ethylsulfonyl)pyrimidin-4-yl)-3-methyl-6-(trifluoromethyl)-3*H*-imidazo[4,5-*b*]pyridine

[Chem. 20]

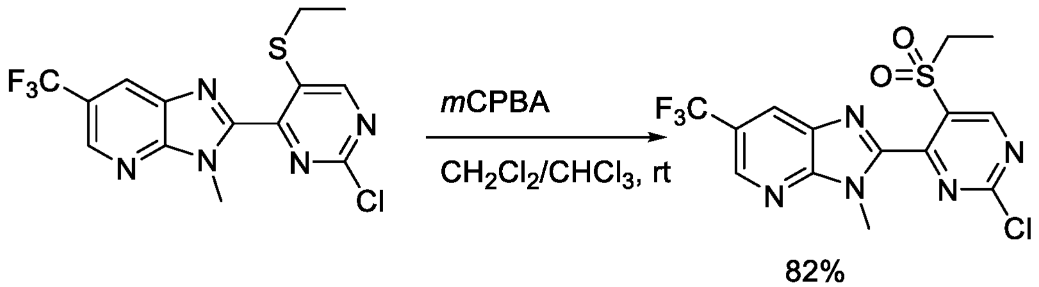


2-(2-chloro-5-(ethylthio)pyrimidin-4-yl)-3-methyl-6-(trifluoromethyl)-3*H*-imidazo[4,5-*b*]pyridine (18.3 g) was dissolved in dichloromethane (300 ml) and chloroform (750 ml), and the mixture was stirred at 0°C. Metachloroperbenzoic acid (70%, 32.3 g) was added thereto, and the mixture was stirred overnight at room temperature. The resultant liquid was poured into a mixed solution of a saturated aqueous solution of sodium hydrogen carbonate and a saturated aqueous solution of sodium thiosulfate. The mixture was subjected to extraction with dichloromethane. The obtained organic layer was washed with a saturated aqueous solution of sodium chloride, dried over anhydrous magnesium sulfate, and filtered. The filtrate was concentrated under reduced pressure, and the obtained residue was washed with diethyl ether. Thereby, the objective product was obtained in an amount of 18.4 g (yield 82%).

$^1$H-NMR of the objective product obtained is shown below.

$^1$H-NMR (400 MHz, $CDCl_3$): δ 9.37 (s, 1H), 8.83 (d, 1H), 8.37 (d, 1H), 4.09 (s, 3H), 4.05 (q, 2H), 1.46 (t, 3H).

(Step 4)

Synthesis of 2-(5-(ethylsulfonyl)-2-hydrazineylpyrimidin-4-yl)-3-methyl-6-(trifluoromethyl)-3*H*-imidazo[4,5-*b*]pyridine

[Chem. 21]

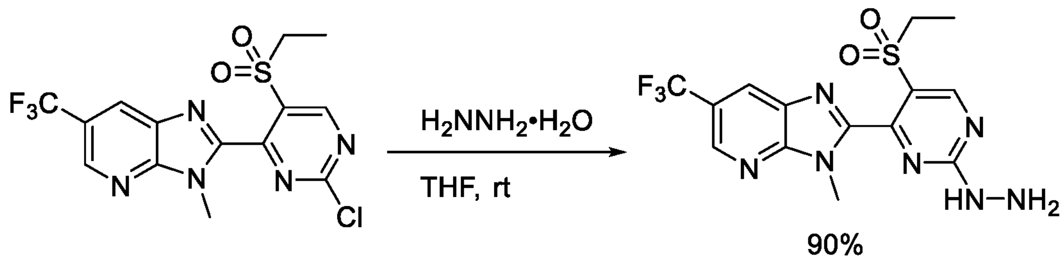


Hydrazine monohydrate (0.50 g) was dissolved in THF (5 ml), and the mixture was stirred at room temperature. 2-(2-Chloro-5-(ethylsulfonyl)pyrimidin-4-yl)-3-methyl-6-(trifluoromethyl)-3*H*-imidazo[4,5-*b*]pyridine (0.41 g) was added thereto, and the mixture was stirred for one hour at room temperature. The resultant liquid was poured into water, and the mixture was subjected to extraction with ethyl acetate. The obtained organic layer was washed with a saturated aqueous solution of sodium chloride, dried over anhydrous magnesium sulfate, and filtered. The filtrate was concentrated under reduced pressure, and the obtained residue was washed with diethyl ether. Thereby, the objective product was obtained in an amount of 0.36 g (yield 90%).

$^1$H-NMR of the objective product obtained is shown below.

$^1$H-NMR (400 MHz, DMSO-$d_6$, 80°C): δ 9.63 (br s, 1H), 8.85 (s, 1H), 8.79 (s, 1H), 8.58 (s, 1H), 4.62 (brs, 2H), 3.88 (s, 3H), 3.62 (q, 2H), 1.22 (t, 3H).

(Step 5)

Synthesis of 1-(5-(ethylsulfonyl)-4-(3-methyl-6-(trifluoromethyl)-3*H*-imidazo[4,5-*b*]pyridin-2-yl)pyrimidin-2-yl)-1*H*-1,2,4-triazol-5-amine

[Chem. 22]

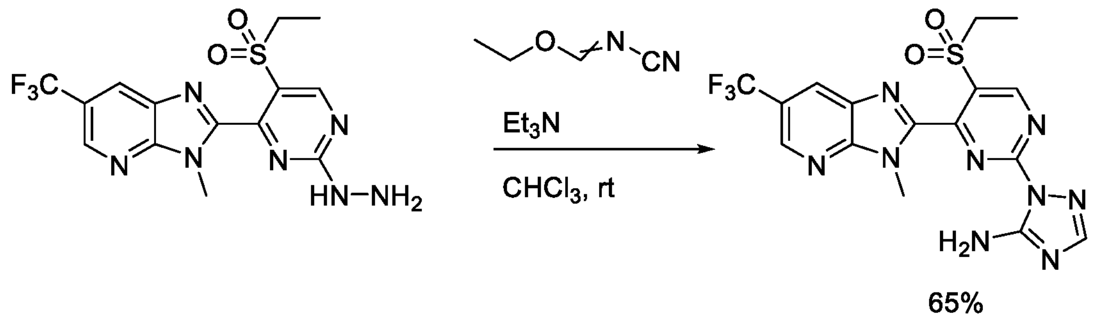


2-(5-(ethylsulfonyl)-2-hydrazineylpyrimidin-4-yl)-3-methyl-6-(trifluoromethyl)-3*H*-imidazo[4,5-*b*]pyridine (0.15 g) was dissolved in chloroform (7 ml), and the mixture was stirred at room temperature. Ethyl *N*-cyanoformimidate (0.05 g) and trimethylamine (0.06 g) were added thereto, and the mixture was stirred overnight at room temperature. The resultant liquid was concentrated under reduced pressure. The obtained residue was purified by column chromatography with silica gel. Thereby, the objective product was obtained in an amount of 0.11 g (yield 65%).

$^1$H-NMR of the objective product obtained is shown below.

$^1$H-NMR (400 MHz, $CDCl_3$): δ 9.46 (s, 1H), 8.83 (d, 1H), 8.37 (d, 1H), 7.72 (s, 1H), 6.75 (br s, 2H), 4.12 (s, 3H), 4.02 (q, 2H), 1.47 (t, 3H).

[Example 6]

Synthesis of ethyl 5-amino-1-(5-(ethylsulfonyl)-4-(3-methyl-6-(trifluoromethyl)-3*H*-imidazo[4,5-*b*]pyridin-2-yl)pyrimidin-2-yl)-1*H*-pyrazole-4-carboxylate (Compound No. A-26)

(Step 1)

Synthesis of ethyl 5-amino-1-(5-(ethylsulfonyl)-4-(3-methyl-6-(trifluoromethyl)-3*H*-imidazo[4,5-*b*]pyridin-2-yl)pyrimidin-2-yl)-1*H*-pyrazole-4-carboxylate

[Chem. 23]

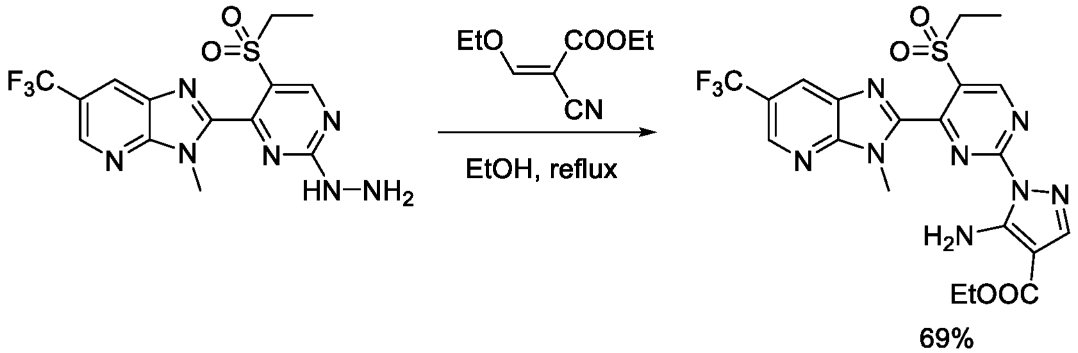


2-(5-(ethylsulfonyl)-2-hydrazinylpyrimidin-4-yl)-3-methyl-6-(trifluoromethyl)-3*H*-imidazo[4,5-*b*]pyridine (0.30 g) was dissolved in ethanol (15 ml), and the mixture was stirred at room temperature. Ethyl (Z)-2-cyano-3-ethoxyacrylate (0.14 g) was added thereto, and the mixture was stirred for 3 hours under refluxing. The resultant liquid was concentrated under reduced pressure, and the obtained residue was purified by column chromatography with silica gel. Thereby, the objective product was obtained in an amount of 0.27 g (yield 69%).

$^1$H-NMR of the objective product obtained is shown below.

$^1$H-NMR (400 MHz, $CDCl_3$) δ 9.44 (s, 1H), 8.82 (d, 1H), 8.36 (d, 1H), 7.94 (s, 1H), 7.51 (br s, 2H), 4.33 (q, 2H), 4.10 (s, 3H), 3.99 (q, 2H), 1.46 (t, 3H), 1.38 (t, 3H).

[Example 7]

Synthesis of 1-(5-(ethylsulfonyl)-4-(3-methyl-6-(trifluoromethyl)-3*H*-imidazo[4,5-*b*]pyridin-2-yl)pyrimidin-2-yl)-1*H*-pyrazol-5-amine (Compound No. A-27)

(Step 1)

Synthesis of 1-(5-(ethylsulfonyl)-4-(3-methyl-6-(trifluoromethyl)-3*H*-imidazo[4,5-*b*]pyridin-2-yl)pyrimidin-2-yl)-1*H*-pyrazol-5-amine

[Chem. 24]

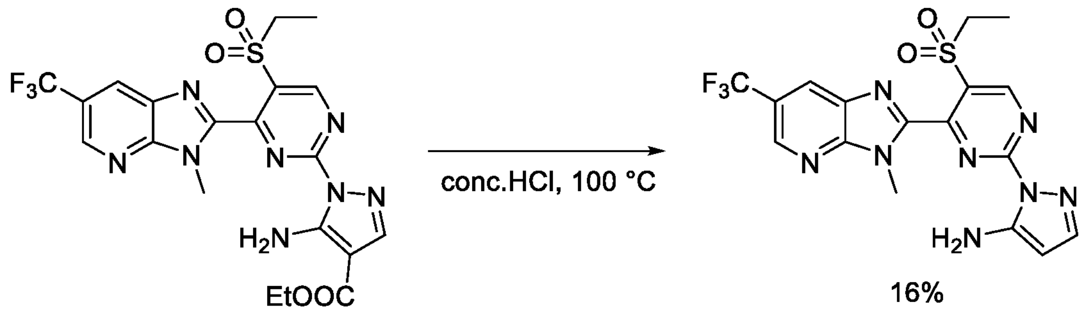


Ethyl 5-amino-1-(5-(ethylsulfonyl)-4-(3-methyl-6-(trifluoromethyl)-3*H*-imidazo[4,5-*b*]pyridin-2-yl)pyrimidin-2-yl)-1*H*-pyrazole-4-carboxylate (0.16 g) was dissolved in concentrated hydrochloric acid (3 ml), and the mixture was stirred for 7 hour at 100°C. The resultant liquid was poured into a saturated aqueous solution of sodium hydrogen carbonate, and the mixture was subjected to extraction with ethyl acetate. The obtained organic layer was washed with a saturated aqueous solution of sodium chloride, dried over anhydrous magnesium sulfate, and filtered. The filtrate was concentrated under reduced pressure, and the obtained residue was purified by column chromatography with silica gel. Thereby, the objective product was obtained in an amount of 0.02 g (yield 16%).

$^1$H-NMR of the objective product obtained is shown below.

$^1$H-NMR (400 MHz, $CDCl_3$): δ 9.39 (s, 1H), 8.80 (d, 1H), 8.36 (d, 1H), 7.60 (d, 1H), 5.89 (br s, 2H), 5.57 (d, 1H), 4.09 (s, 3H), 3.97 (q, 2H), 1.45 (t, 3H).

Examples of the compounds according to the present invention prepared by the same methods as those described in the aforementioned Examples are shown in Table 1. Table 1 shows the substituents of the compounds represented by Formula (I). In the tables, Me represents a methyl group, Et represents an ethyl group, iPr represents an isopropyl group, Bz represents a benzoyl group, Ac represents an acetyl group, and Ph represents a phenyl group. In addition, the physical data of the compounds are described in the columns of "Physical property". As the physical property data, the melting points (m. p.) are described. With respect to the compounds indicated with *, the NMR data are shown.

[Chem. 25]

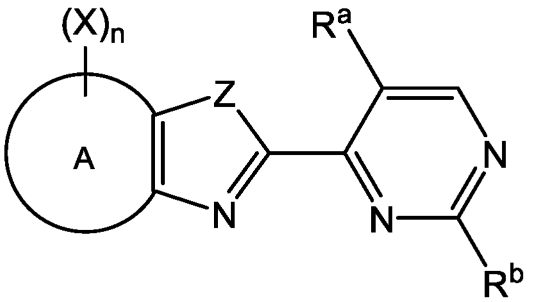

(I)

Table 1

| Compound No. | 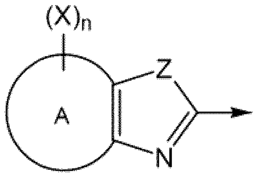 | $R^a$ | $R^b$ | Physical property |
|---|---|---|---|---|
| A-1 | 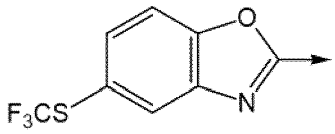 | $SO_2Et$ | OMe | m.p. 226 - 228°C |
| A-2 | 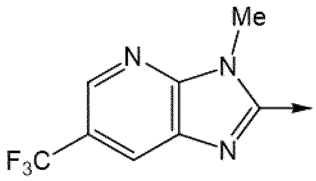 | $SO_2Et$ | pyrimidin-2-yl | m.p. 273 - 275°C |
| A-3 | 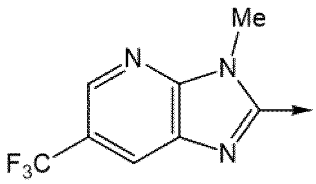 | $SO_2Et$ | 1-($CH_2OCH_2CH_2$-$SiMe_3$)-1H-1,2,4-triazol-3-yl | m.p. 237 - 240°C |
| A-4 | 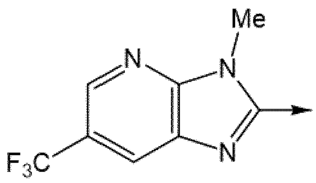 | $SO_2Et$ | 1H-1,2,4-triazol-3-yl | m.p. 294 - 296°C |
| A-5 | 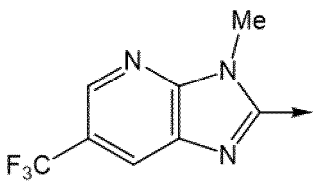 | $SO_2Et$ | 1-Me-1H-1,2,4-triazol-3-yl | m.p. 280°C |
| A-6 | 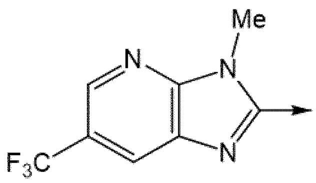 | $SO_2Et$ | 1-($CH_2CF_3$)-1H-1,2,4-triazol-3-yl | * |
| A-7 | 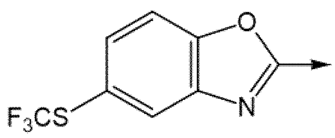 | $SO_2Et$ | pyrimidin-2-yl | m.p. 223 - 225°C |
| A-8 | 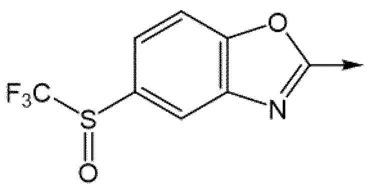 | $SO_2Et$ | pyrimidin-2-yl | m.p. 192 - 195°C |

(continued)

| Compound No. | 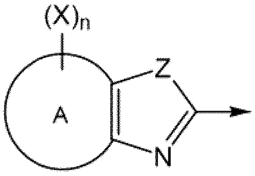 | $R^a$ | $R^b$ | Physical property |
|---|---|---|---|---|
| A-9 | 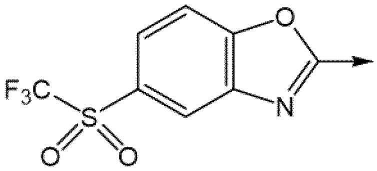 | $SO_2Et$ | pyrimidin-2-yl | m.p. 118 - 124°C |
| A-10 | 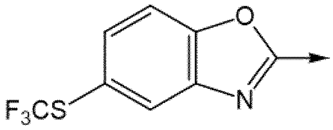 | $SO_2Et$ | $NH_2$ | m.p. 218 - 224°C |
| A-11 | 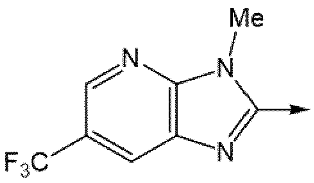 | $SO_2Et$ | 3-$NH_2$-1H-1,2,4-triazol-1-yl | m.p. 264 - 266°C |
| A-12 | 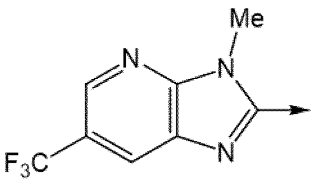 | $SO_2Et$ | 5-$NH_2$-1H-1,2,4 -triazol-1-yl | m.p. 275 - 277°C |
| A-13 | 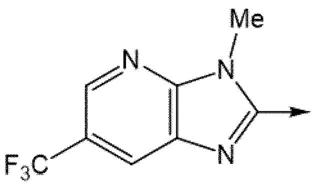 | $SO_2Et$ | $NH_2$ | m.p. 260 - 262°C |
| A-14 | 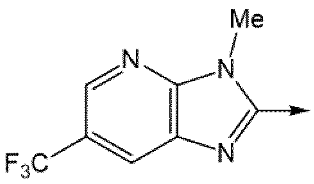 | $SO_2Et$ | $NMeNH_2$ | m.p. 163 - 165°C |
| A-15 | 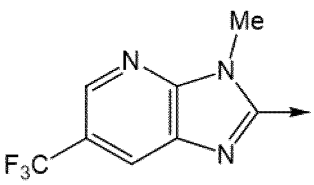 | $SO_2Et$ | NHC(=O)NHMe | m.p. 229 - 23 1°C |

(continued)

| Compound No. | 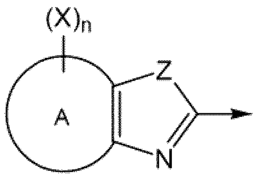 | $R^a$ | $R^b$ | Physical property |
|---|---|---|---|---|
| A-16 | 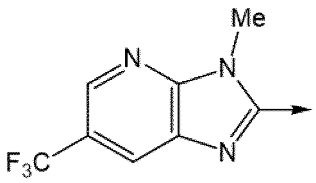 | $SO_2Et$ | NHMe | m.p. 228 - 230°C |
| A-17 | 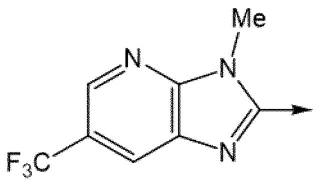 | $SO_2Et$ | $NMe_2$ | m.p. 179 - 180°C |
| A-18 | 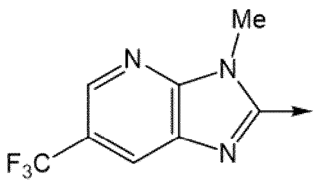 | $SO_2Et$ | NMeAc | m.p 200 - 201°C |
| A-19 | 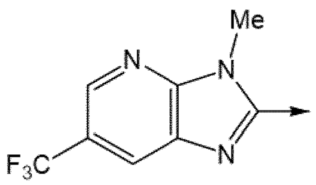 | $SO_2Et$ | 1H-1,2,4-triazol-1-yl | m.p. 275 - 277°C |
| A-20 | 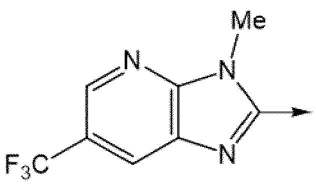 | $SO_2Et$ | 1H-pyrazol-1-yl | m.p 202 - 205°C |
| A-21 | 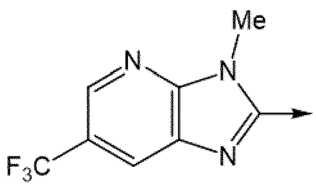 | $SO_2Et$ | 1H-imidazol-1-yl | m.p. 218 - 220°C |
| A-22 | 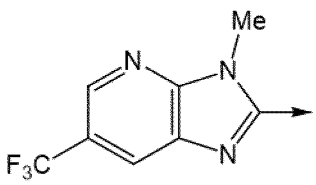 | $SO_2Et$ | 4-$NH_2$-1H-pyrazol-1-yl | m.p. 155 - 159°C |

(continued)

| Compound No. | 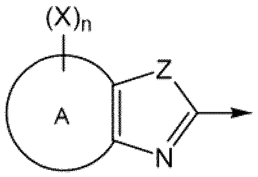 | $R^a$ | $R^b$ | Physical property |
|---|---|---|---|---|
| A-23 | 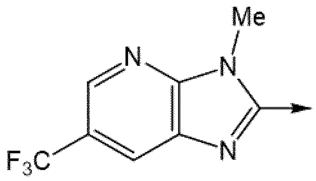 | $SO_2Et$ | OMe | m.p. 151 - 152°C |
| A-24 | 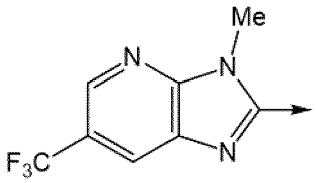 | $SO_2Et$ | $NHNH_2$ | m.p. 227 - 229°C |
| A-25 | 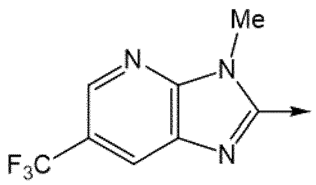 | $SO_2Et$ | 3-NH2-1H-pyrazol-1-yl | m.p. 278 - 280°C |
| A-26 | 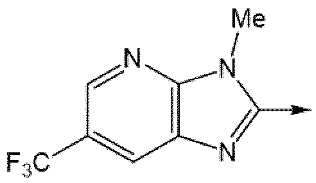 | $SO_2Et$ | 5-$NH_2$-4-COOEt-1H-pyrazol-1-yl | m.p. 233 - 234°C |
| A-27 | 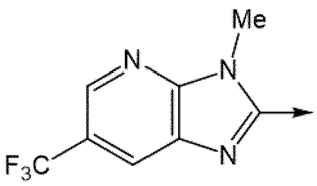 | $SO_2Et$ | 5-NH2-1H-pyrazol-1-yl | m.p. 247 - 249°C |
| A-28 | 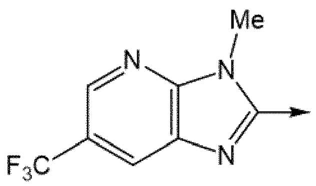 | $SO_2Et$ | $NHCH_2CH_2NH_2$ | m.p. 166 - 167°C |
| A-29 | 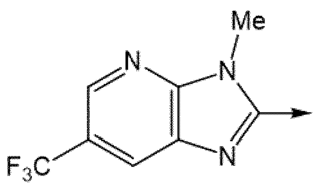 | $SO_2Et$ | $NHC(=NH)NH_2$ | m.p. 265 - 268°C |

(continued)

| Compound No. | 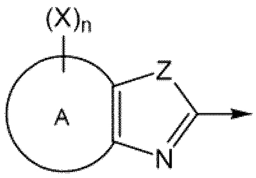 | $R^a$ | $R^b$ | Physical property |
|---|---|---|---|---|
| A-30 | 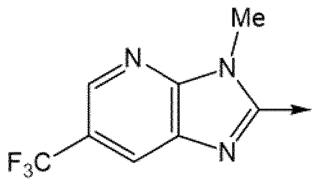 | $SO_2Et$ | $OCH_2CF_3$ | m.p. 171 - 172°C |
| A-31 | 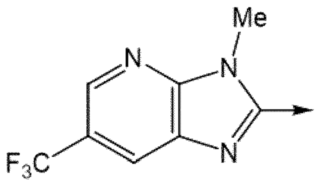 | $SO_2Et$ | OPh | m.p. 172 - 173°C |
| A-32 | 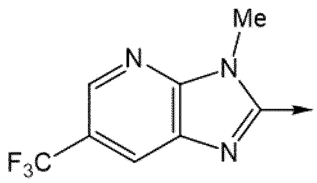 | $SO_2Et$ | $OCH_2CH_2OH$ | m.p. 162 - 164°C |
| A-33 | 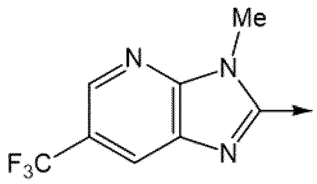 | $SO_2Et$ | NHC(=NH)OEt | m.p. 202 - 205°C |
| A-34 | 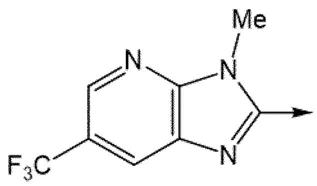 | $SO_2Et$ | 5-$NH_2$-4-CN-1H-pyrazol-1-yl | m.p. 290 - 294°C |
| A-35 | 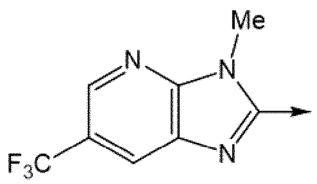 | $SO_2Et$ | 4-COOEt-1H-pyrazol-1-yl | m.p. 169 - 172°C |
| A-36 | 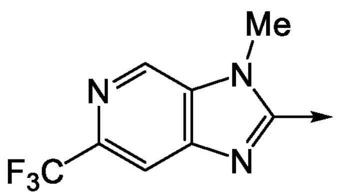 | $SO_2Et$ | $NHNH_2$ | m.p. 291 - 293°C |

(continued)

| Compound No. | 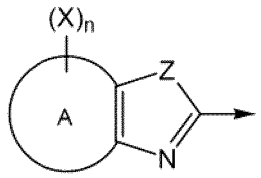 | $R^a$ | $R^b$ | Physical property |
|---|---|---|---|---|
| A-37 | 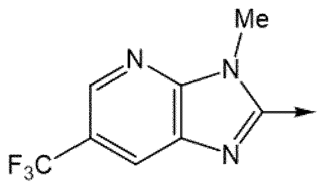 | $SO_2Et$ | 5-$NH_2$-4-$COO^iPr$-1H-pyrazol-1-yl | m.p. 224 - 227°C |
| A-38 | 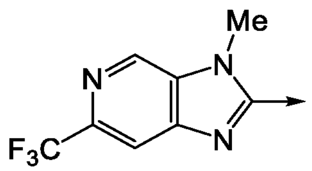 | $SO_2Et$ | 5-$NH_2$-1H-1,2,4-triazol-1-yl | m.p. 270 - 274°C |
| A-39 | 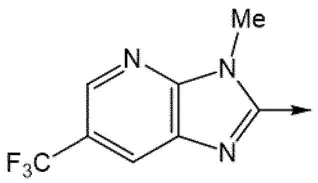 | $SO_2Et$ | 5-$NH_2$-4-Bz-1H-pyrazol-1-yl | m.p. 166 - 169°C |
| A-40 | 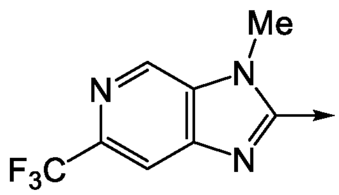 | $SO_2Et$ | pyrimidin-2-yl | m.p. 258 - 260°C |
| A-41 | 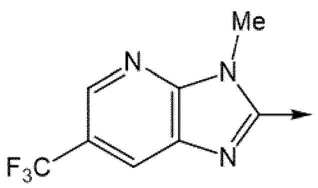 | $SO_2Et$ | 3-COOEt-1H-pyrazol-1-yl | m.p. 204 - 206°C |
| A-42 | 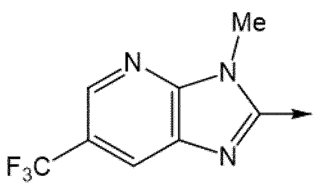 | $SO_2Et$ | 5-$NH_2$-4-$SO_2Me$-1H-pyrazol-1-yl | m.p. 167 - 171°C |
| A-43 | 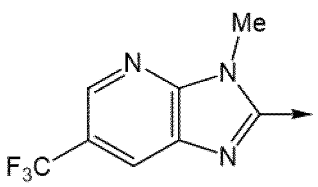 | $SO_2Et$ | 5-$NH_2$-4-Ac-1H-pyrazol-1-yl | m.p. 236 - 240°C |

The NMR data of Compound No. A-6 are shown below.

$^1H$-NMR ($CDCl_3$) $\delta$: 9.63 (1H, s), 8.81 (1H, d), 8.46 (1H, s), 8.36 (1H, d), 4.99 (2H, q), 4.11 (3H, s), 4.03 (2H, q), 1.47 (3H, t).

[Biological tests]

The following Test Examples demonstrate that the compounds of the present invention are useful as active ingredients of the formulations for controlling harmful organisms, and of the formulations for controlling ectoparasites. The term "part" is based on weight.

(Preparation of emulsion for test)

5 parts of the compound of the present invention, 93.6 parts of dimethylformamide and 1.4 parts of polyoxyethylene alkyl aryl ether were mixed and dissolved to prepare Emulsion (I) including 5% of an active ingredient.
For the control, Emulsion (II) was prepared by mixing and dissolving 93.6 parts of dimethylformamide and 1.4 parts of polyoxyethylene alkyl aryl ether.
An insect mortality rate was calculated by the numerical equation shown below.

$$\text{Insect mortality rate (\%)} =$$

$$\{(\text{Number of dead insects}) / (\text{Number of sample insects})\} \times 100$$

(Test Example 1) Efficacy Test against *Mythimna separata*

0.8 g of a commercially available artificial feed (Insecta LFS, manufactured by Nosan Corporation) and 1 μl of Emulsion (I) were mixed thoroughly. Thereby, a test feed was obtained.
The test feed was placed in an amount of 0.2 g in each of the treatment areas of a plastic test container (volume: 1.4 ml). Subsequently, two second-instar larvae of *Mythimna separata* were inoculated into each treatment area, and the test container was sealed with a plastic lid in order to prevent the larvae from escaping. The sealed container was placed in a thermostatic chamber at 25°C, and the insect mortality rate and the amount of feed consumed were determined on the fifth day. The test was performed twice.
As the control areas, the insect mortality rate and the amount of feed consumed were determined in the same manner as that described in Test Example 1, with the exception of replacing Emulsion (I) with Emulsion (II).
Efficacy tests against *Mythimna separata* were conducted for Compound Nos. A-2, A-5, A-10, A-12, A-13, A-16, A-17, A-20, A-21, A-23, A-25, A-26, A-28, A-37, A-39, and A-43. For all of the compounds mentioned above, the mortality rate against *Mythimna separata* was 100%, or the amount of feed consumed was 10% or less of the amount of feed consumed in the control area.

(Test Example 2) Efficacy test against *Plutella xylostella*

Emulsion (I) was diluted with water so that the concentration of the compound of the present invention was 125 ppm. Cabbage leaves were soaked in the diluted liquid for 30 seconds. Subsequently, the cabbage leaves were put on Petri dishes, followed by inoculating 5 second-instar larvae of *Plutella xylostella.* The Petri dishes were placed in a thermostatic chamber at a temperature of 25°C and humidity of 60%. Mortality was investigated 3 days after inoculation, and the insect mortality rate was calculated. The test was performed twice.
The efficacy test against *Plutella xylostella* was carried out for Compound Nos. A-2, A-5, A-6, A-12, A-15, A-23, A-26, A-30, A-34, A-37, and A-39. All of the compounds mentioned above demonstrated an 80% or more insect mortality rate against *Plutella xylostella.*

(Test Example 3) Efficacy test against *Aphis craccivora*

Black-eyed pea plants were raised in No. 3 pots and the primary leaves were inoculated with nymphs of *Aphis craccivora.* Emulsion (I) was diluted with water so that the concentration of the compound of the present invention was 125 ppm. Subsequently, the aforementioned diluted liquid was sprayed on the black-eyed pea plants on which the nymphs of *Aphis craccivora* were parasitic. The aforementioned black-eyed pea plants were then placed in a thermostatic chamber with a temperature of 25°C and humidity of 60%. Mortality was investigated 4 days after spraying was carried out, and the insect mortality rate of *Aphis craccivora* was calculated. The test was performed twice.
The efficacy test against *Aphis craccivora* was carried out for Compound Nos. A-2, A-4, A-5, A-14, A-16, A-20, A-23, A-26, A-33, A-35, A-36, A-37, and A-38. All of the compounds mentioned above demonstrated an 80% or more mortality rate against *Aphis craccivora.*

(Test Example 4) Efficacy test against *Phyllotreta striolata*

Emulsion (I) was diluted with water so that the concentration of the compound of the present invention was 125 ppm, to prepare a diluted liquid for testing. The aforementioned diluted liquid was sprayed on bok-choi seedlings ($7^{th}$ major leaf-development period) planted in No. 3 pots. After the bok-choi seedlings were subjected to air-drying, the seedlings were placed in plastic cups, followed by inoculating 10 adult *Phyllotreta striolata.* The plastic cups were stored in a thermostatic chamber at a temperature of 25°C and humidity of 65%. Mortality was investigated 7 days after inoculation, and the insect mortality rate was calculated. The test was performed twice.

The efficacy test against adult *Phyllotreta striolata* was carried out for Compound Nos. A-2, A-5, A-6, A-11, A-12, A-15, A-20, A-26, A-38, A-42, and A-43. All of the compounds demonstrated an 80% or more mortality rate against adult *Phyllotreta striolata.*

(Test Example 5) Efficacy test against *Nilaparvata lugens*

Emulsion (I) was diluted with water so that the concentration of the compound of the present invention was 125 ppm. Rice seedlings were soaked in the diluted liquid for 30 seconds, and subjected to air-drying. Subsequently, the rice seedlings were placed in plastic cases, followed by inoculating 5 second-instar larvae of *Nilaparvata lugens.* The plastic cases were placed in a thermostatic chamber at a temperature of 25°C and humidity of 65%. Mortality was investigated 7 days after inoculation, and the insect mortality rate was calculated. The test was performed twice.

The efficacy test against *Nilaparvata lugens* was carried out for Compound Nos. A-2, A-4, A-5, A-11, A-12, A-15, A-16, A-18, A-20, A-23, A-34, A-38, A-40, A-42, and A-43. All of the aforementioned compounds demonstrated an 80% or more mortality rate against *Nilaparvata lugens.*

The compounds selected at random among the heteroarylpyrimidine compounds according to the present invention exhibit the effects described above. For this reason, it can be understood that the heteroarylpyrimidine compounds of the present invention including those which cannot be demonstrated above have effects of controlling harmful organisms, and in particular, acaricidal effects, insecticidal effects and the like. In addition, it can also be understood that the heteroarylpyrimidine compounds of the present invention have effects on ectoparasites and the like which harm humans and animals.

INDUSTRIAL APPLICABILITY

Heteroarylpyrimidine compounds of the present invention can control harmful organisms which are problematic in view of farm products or for hygiene reasons. In particular, the heteroarylpyrimidine compounds can effectively control agricultural pests and acari with a reduced concentration. In addition, the heteroarylpyrimidine compounds of the present invention can effectively control ectoparasites and endoparasites which harm humans and animals. The formulation for controlling harmful organisms containing the compounds of the present invention as an active ingredient can be utilized as, for example, an insecticidal formulation or an acaricidal formulation, a formulation for controlling ectoparasites, and/or a formulation for controlling endoparasites or for expelling endoparasites.

**Claims**

1. A compound represented by Formula (I), an N-oxide compound thereof, a stereoisomer, a tautomer, a hydrate, or a salt thereof,

[Chem. 1]

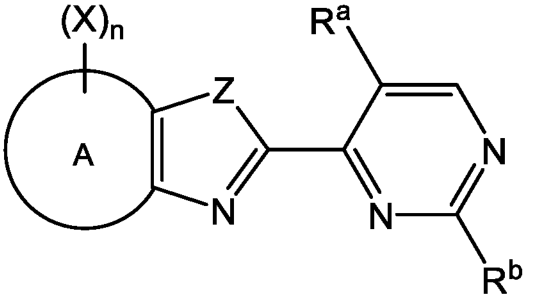

(I)

wherein

X is a halogeno group, a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a nitro group, a cyano group, or a group represented by $-NR^1R^2$,
each of $R^1$ and $R^2$ is independently a hydrogen atom, a substituted or unsubstituted C1-6 alkyl group, a substituted or unsubstituted C1-6 alkyl carbonyl group, or a substituted or unsubstituted C1-6 alkoxy carbonyl group,
n is an integer of any one of 1 to 3,
Z is an oxygen atom, a sulfur atom or NR,
R is a hydrogen atom or a substituted or unsubstituted C1-6 alkyl group,
A is a benzene ring or a 6-membered heteroaryl ring,
$R^a$ is a substituted or unsubstituted C1-6 alkylsulfonyl group,
$R^b$ is a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkyl carbonyl group, a substituted or unsubstituted C1-6 alkoxy carbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a nitro group, a cyano group, a group represented by $-NR^3R^4$, a group represented by $-C(=O)NR^8R^9$, a group represented by $-C(=NH)R^{10}$, a group represented by $-CR^{11}=N-OR^{12}$, a group represented by-$C(=O)N=S(O)_mR^{13}$, or a group represented by $-N=S(O)R^{14}R^{15}$,
each of $R^3$ and $R^4$ is independently a hydrogen atom, a substituted or unsubstituted C1-6 alkyl group, a substituted or unsubstituted C1-6 alkyl carbonyl group, a substituted or unsubstituted C1-6 alkoxy carbonyl group, an amino group, a group represented by $-C(=O)NR^5R^6$, or a group represented by $-C(=NH)R^7$,
each of $R^5$ and $R^6$ is independently a hydrogen atom, or a substituted or unsubstituted C1-6 alkyl group,
$R^7$ is a C1-6 alkoxy group, or an amino group,
each of $R^8$ and $R^9$ is independently a hydrogen atom, or a substituted or unsubstituted C1-6 alkyl group,
$R^{10}$ is a C1-6 alkoxy group, or an amino group,
each of $R^{11}$ and $R^{12}$ is independently a hydrogen atom, or a substituted or unsubstituted C1-6 alkyl group,
m is 0 or 1,
$R^{13}$ is a C1-6 alkyl group, and
each of $R^{14}$ and $R^{15}$ is a C1-6 alkyl group.

**2.** A formulation for controlling harmful organisms, comprising at least one compound selected from the group consisting of the compounds as recited in Claim 1, N-oxide compounds thereof, stereoisomers, tautomers, hydrates, and salts thereof, as an active ingredient.

**3.** An insecticidal formulation or an acaricidal formulation, comprising at least one compound selected from the group consisting of the compounds as recited in Claim 1, N-oxide compounds thereof, stereoisomers, tautomers, hydrates, and salts thereof, as an active ingredient.

**4.** A formulation for controlling ectoparasites, comprising at least one compound selected from the group consisting of the compounds as recited in Claim 1, N-oxide compounds thereof, stereoisomers, tautomers, hydrates, and salts thereof, as an active ingredient.

**5.** A formulation for controlling endoparasites or for expelling endoparasites, comprising at least one compound selected from the group consisting of the compounds as recited in Claim 1, N-oxide compounds thereof, stereoisomers, tautomers, hydrates, and salts thereof, as an active ingredient.

## INTERNATIONAL SEARCH REPORT

International application No. PCT/JP2019/009418

A. CLASSIFICATION OF SUBJECT MATTER

Int.Cl. C07D471/04(2006.01)i, A01N43/76(2006.01)i, A01N43/90(2006.01)i, A01N47/02(2006.01)i, A01P7/04(2006.01)i, A61K31/506(2006.01)i, A61P33/00(2006.01)i, A61P33/14(2006.01)i, C07D413/04(2006.01)i, C07D413/14(2006.01)i, C07F7/10(2006.01)i

According to International Patent Classification (IPC) or to both national classification and IPC

B. FIELDS SEARCHED

Minimum documentation searched (classification system followed by classification symbols)

Int.Cl. C07D471/04, A01N43/76, A01N43/90, A01N47/02, A01P7/04, A61K31/506, A61P33/00, A61P33/14, C07D413/04, C07D413/14, C07F7/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

CAplus/REGISTRY/Marpat (STN)

C. DOCUMENTS CONSIDERED TO BE RELEVANT

| Category* | Citation of document, with indication, where appropriate, of the relevant passages | Relevant to claim No. |
|---|---|---|
| Y | WO 2017/025419 A2 (BAYER CROPSCIENCE AKTIENGESELLSCHAFT) 16 February 2017, claims, page 176, lines 1-9, page 184, line 1 to page 222, line 26 & JP 2018-523664 A & US 2018/0271099 A1, claims, paragraphs [0896]-[0897], [0967]-[1196] & EP 3331870 A2 & KR 10-2018-0032640 A & CN 108137548 A | 1-5 |

☒ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 16 May 2019 (16.05.2019) | 28 May 2019 (28.05.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2019/009418

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2016/124557 A1 (BAYER CROPSCIENCE AKTIENGESELLSCHAFT) 11 August 2016, claims, page 127, lines 1-9, page 135, line 1 to page 171, line 12 & JP 2018-509391 A & US 2018/0016273 A1, claims, paragraphs [0737]-[0738], [0772]-[0958] & EP 3253757 A1 & KR 10-2017-0113600 A & CN 107428759 A | 1-5 |
| Y | JP 2013-136519 A (SUMITOMO CHEMICAL CO., LTD.) 11 July 2013, claims, paragraphs [0378], [0543], [0684], [0689], table 20 & US 2014/0364444 A1 & WO 2012/086848 A1, claims, paragraphs [0283], [0459], [0599], [0604], table 20 & EP 2655337 A1 & CN 103261170 A & KR 10-2013-0140125 A | 1-5 |
| Y | JP 2014-005263 A (SUMITOMO CHEMICAL CO., LTD.) 16 January 2014, claims, paragraphs [0262], [0398], [0476], [0479], table 36 & US 2014/0194290 A1 & WO 2013/018928 A1, claims, paragraphs [0270], [0398], [0471], [0474], table 36 & EP 2739624 A1 & CN 103717598 A & KR 10-2014-0068920 A | 1-5 |
| Y | JP 2012-131780 A (SUMITOMO CHEMICAL CO., LTD.) 12 July 2012, claims, paragraphs [0076], [0120], [0134]-[0135], table 12 & US 2013/0252981 A1 & WO 2012/074135 A1, claims, paragraphs [0071], [0116], [0130]-[0131], table 12 & EP 2646433 A1 & CN 103228646 A | 1-5 |
| A | JP 2016-528189 A (SYNGENTA PARTICIPATIONS AG.) 15 September 2016, entire text & US 2016/0255837 A1 & WO 2015/000715 A1, entire text & EP 3016949 A1 & CN 105431433 A & KR 10-2016-0029078 A | 1-5 |
| A | WO 2016/039441 A1 (NIHON NOHYAKU CO., LTD.) 17 March 2016, entire text & TW 201625522 A | 1-5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018044222 A
- WO 2016121969 A
- WO 2017069105A PCT
- WO 2012086848 A
- WO 2011049222 A


### Non-patent literature cited in the description


- **COLBY. S. R.** Calculating Synergistic and Antagonistic Responses of Herbicide Combinations. *Weeds,* 1967, vol. 15, 20-22